(19) Europäisches Patentamt / European Patent Office / Office européen des brevets

(11) **EP 3 976 692 B1**

(12) **EUROPÄISCHE PATENTSCHRIFT**

(45) Veröffentlichungstag und Bekanntmachung des Hinweises auf die Patenterteilung:
**22.02.2023   Patentblatt 2023/08**

(21) Anmeldenummer: **19728036.5**

(22) Anmeldetag: **29.05.2019**

(51) Internationale Patentklassifikation (IPC):
**C08G 77/388** (2006.01)   **C08G 77/452** (2006.01)
**C08G 77/54** (2006.01)   **C08K 5/11** (2006.01)
**C08L 83/08** (2006.01)   **C08L 83/10** (2006.01)
**C08L 83/14** (2006.01)

(52) Gemeinsame Patentklassifikation (CPC):
(C-Sets verfügbar)
**C08G 77/54; C08G 77/388; C08G 77/452;**
**C08K 5/11; C08L 83/08; C08L 83/10; C08L 83/14;**
C08G 77/26                                              (Forts.)

(86) Internationale Anmeldenummer:
**PCT/EP2019/064120**

(87) Internationale Veröffentlichungsnummer:
**WO 2020/239229 (03.12.2020 Gazette 2020/49)**

(54) **WÄSSRIGE DISPERSIONEN VON VORVERNETZTEN ORGANOPOLYSILOXANEN**

AQUEOUS DISPERSIONS OF PRE-CROSSLINKED ORGANOPOLYSILOXANES

DISPERSIONS AQUEUSES D'ORGANOPOLYSILOXANES PRÉRÉTICULÉS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**06.04.2022   Patentblatt 2022/14**

(73) Patentinhaber: **Wacker Chemie AG**
**81737 München (DE)**

(72) Erfinder:
• **GRANDL, Markus**
**81825 München (DE)**
• **BEER, Gerhard**
**84489 Burghausen (DE)**
• **LIMMER, Werner**
**84568 Pleiskirchen (DE)**
• **SELBERTINGER, Ernst**
**84489 Burghausen (DE)**

(74) Vertreter: **Mieskes, Klaus Theoderich**
**Wacker Chemie AG**
**Intellectual Property**
**Hanns-Seidel-Platz 4**
**81737 München (DE)**

(56) Entgegenhaltungen:
WO-A1-2015/024079     US-A1- 2008 318 057
US-A1- 2013 012 667     US-B2- 8 609 787

(52) Gemeinsame Patentklassifikation (CPC): (Forts.)

C-Sets
**C08K 5/11, C08L 83/08**

**Beschreibung**

Die Erfindung betrifft wässrige Dispersionen von vorvernetzten Organopolysiloxanen, vorvernetzte Organopolysiloxane und kosmetische Zusammensetzungen enthaltend wässrige Dispersionen von vorvernetzten Organopolysiloxanen. Weiterhin betrifft die Erfindung Verfahren zur Herstellung der vorvernetzten Organopolysiloxane und deren wässrige Dispersionen. Weiterhin betrifft die Erfindung die Verwendung der kosmetischen Zusammensetzungen.

Organopolysiloxane werden in kosmetischen Zusammensetzungen, beispielsweise bei Haarpflegeprodukten, verwendet aufgrund ihrer konditionierenden Eigenschaften, wie Verbesserung von Weichheit und Glätte, Reduzierung von Kämmkräften, Glanzeigenschaften, Verbesserung von Farbeindrücken, Farbschutzeigenschaften, Verringerung der elektrostatischen Aufladungen, Schutzeigenschaften bei thermischer Belastung des Haars oder Hydrophobierung.

Eine Übersicht über ausgewählte Organopolysiloxane für Pflege keratinischer Materialien wie Haare findet sich in M.D. Berthiaume, Society of Cosmetic Chemists (Hrsg.), Monograph, Silicones in Hair Care, 1997 und J. Sejpka, Silicone in Haarpflegeprodukten, in: SÖFW-Journal, 118. Jahrgang, Nr. 17, 1992, S. 1065-1070.

Haare sind im Alltag einer Vielzahl von äußeren Einflüssen ausgesetzt, die zur Schädigung der Haaroberfläche führen, und damit die kosmetischen Eigenschaften wie Glätte, Weichheit, Glanz und andere Parameter im Vergleich zu ungeschädigtem Haar verschlechtern. Die Schädigung der Haaroberfläche kann beispielsweise durch chemische oder mechanische Behandlung verursacht werden, durch UV-Strahlung oder durch Hitze. Einhergehend mit der Oberflächenschädigung von Haar ist die Zerstörung und teilweise Entfernung der die Cuticula bedeckenden Lipidschicht, die ursächlich für die stark hydrophobe Eigenschaft von ungeschädigtem, natürlichem Haar ist (R.A. Lodge, B. Bhusan, Wetting Properties of Human Hair by Means of Dynamic Contact Angle Measurement, Journal of Applied Polymer Science, Vol. 102, 5255-5265, 2006, Wiley). Geschädigtes Haar ist im Vergleich zu ungeschädigtem Haar deutlich hydrophiler, da nach Zerstörung der oberflächlichen Lipidschicht eine hydrophile, aminosäurebasierte Proteinmatrix als Haaroberfläche wirkt.

In US 7,223,385 B2, US 7,485,289 B2, US 7,220,408 B2 und US 7,504,094 B2 werden kosmetische Zusammensetzungen zur Behandlung von Haaren beschrieben, die spezielle Aminosilicone der Struktur (I) oder (II) enthalten sowie ein Konditionierungsmittel oder einen Verdicker. Bei den Aminosiliconen der Struktur (I) oder (II) handelt es sich um endständige Alkoxy-/Hydroxy-gruppen aufweisende Dimethylpolysiloxane mit Aminoethylaminopropyl-Alkoxy-Siloxaneinheiten oder Aminoethylaminopropyl-Methyl-Siloxaneinheiten, die unvernetzt sind.

Aus US 5,039,738 A ist ein Verfahren zur Herstellung modifizierter Aminoorganosiloxane bekannt, bei dem Aminoorganosiloxane in wässrigen Emulsionen mit Dialkyloxalaten, Dialkylpyrocarbonaten oder deren Mischung umgesetzt werden. Es zeigte sich, dass sich textile Stoffe, welche mit den beschriebenen Emulsionen behandelt wurden, durch verringerte Vergilbung auszeichnen.

In WO 2015/024079 A1 werden kosmetische Zusammensetzungen beschrieben, die Aminoorganopolysiloxane, kationische Tenside und Dialkyldicarbonsäureester der Formel R'-O-CO-R-CO-O-R' beinhalten, wobei R' C8-C30-Rest beinhaltet.

Gemäß WO 2004/039930 A2 zeigen Textilien, die mit einer Zusammensetzung aus Polycarbonsäuren und Aminoorganosiloxanen behandelt wurden, eine verbesserte Kräusel- und Faltenbeständigkeit.

Eine vernetzbare Zusammensetzung aus Aminoorganopolysiloxan und einer Vernetzerkomponente, welche ein mindestens eine Carbonsäureanhydridgruppe aufweisendes Alkoxysilan oder Siloxan ist, wird in US 5,399,652 A beschrieben.

US 8 609 787 B2 offenbart die Umsetzung eines Diamino-terminierten Polysiloxans mit Ethyloxalat zur Herstellung eines Polyamid-verknüpften Polysiloxans.

Es bestand die Aufgabe, vorvernetze Organopolysiloxane, insbesondere wässrige Dispersionen von vorvernetzten Organopolysiloxanen, bereitzustellen, die vorzugsweise nach Entfernen des Wassers einen elastomeren Film bilden und die in kosmetischen Zusammensetzungen eingesetzt werden können. Weiterhin bestand die Aufgabe kosmetische Zusammensetzungen zur Behandlung von keratinischen Fasern, wie Haaren, vorzugsweise zu deren Reinigung und Pflege, bereitzustellen, die zum Konditionieren von keratinischen Fasern, wie Haaren, verwendet werden können, insbesondere um deren Kämmbarkeit zu erleichtern.

Die Aufgabe wird durch die Erfindung gelöst.

Gegenstand der Erfindung sind wässrige Dispersionen, vorzugsweise wässrige Emulsionen, enthaltend vorvernetzte Organopolysiloxane, die durchschnittlich mindestens eine Struktureinheit, vorzugsweise mindestens zwei Struktureinheiten, bevorzugt mindestens drei Struktureinheiten, der allgemeinen Formel

$$SiRO_{2/2}\text{-}Y\text{-}SiRO_{2/2} \qquad (I)$$

und Einheiten der Formel

$$R_2SiO_{2/2} \qquad (II),$$

enthalten, wobei

Y ein Rest der Formel

$-R^2-[NR^3-R^4-]_x-NR^3-OC-[C(Z^1)(H)]_{k1}-[C(Z^2)(H)]_{k2}-CO-NR^3-[R^4-NR^3]_x-R^2-$bedeutet,

R gleich oder verschieden sein kann, einen einwertigen Kohlenwasserstoffrest mit 1 bis 18 Kohlenstoffatomen bedeutet, der ein oder mehrere Heteroatome aus der Gruppe N, P, S, O, und Halogen enthalten kann,

$R^2$ einen SiC-gebundenen, zweiwertigen linearen oder verzweigten Kohlenwasserstoffrest mit 3 bis 18 Kohlenstoffatomen, vorzugsweise einen Alkylenrest mit 3 bis 10 Kohlenstoffatomen, bedeutet,

$R^3$ ein Wasserstoffatom, einen Alkylrest mit 1 bis 8 Kohlenstoffatomen oder einen Acylrest, wie Acetylrest, bedeutet, vorzugsweise ein Wasserstoffatom ist,

$R^4$ einen zweiwertigen Kohlenwasserstoffrest mit 1 bis 6 Kohlenstoffatomen, vorzugsweise einen Alkylenrest mit 1 bis 6 Kohlenstoffatomen, bedeutet,

k1 0, 1, 2 oder 3 ist,

k2 0, 1, 2 oder 3 ist,

x 0, 1, 2, 3 oder 4, vorzugsweise 0 oder 1 ist,

$Z^1$ -OH, H oder $-NHR^3$ bedeutet,

$Z^2$ -OH, H oder $-NHR^3$ bedeutet,

mit der Maßgabe, dass die Summe k1+k2$\geq$1 ist und dass mindestens ein Rest $Z^1$ oder $Z^2$ eine Hydroxy- oder $NHR^3$-Gruppe, vorzugsweise eine Hydroxygruppe, ist, in der verbrückenden Gruppe Y damit mindestens eine Hydroxygruppe oder NHR-Gruppe enthalten ist.

Gegenstand der Erfindung sind weiterhin vorvernetzte Organopolysiloxane, die durchschnittlich mindestens eine Struktureinheit, vorzugsweise mindestens zwei Struktureinheiten, bevorzugt mindestens drei Struktureinheiten, der allgemeinen Formel

$$SiRO_{2/2}-Y-SiRO_{2/2} \qquad (I)$$

und Einheiten der Formel

$$R_2SiO_{2/2} \qquad (II),$$

enthalten, wobei

Y ein Rest der Formel

$-R^2-[NR^3-R^4-]_x-NR^3-OC-[C(Z^1)(H)]_{k1}-[C(Z^2)(H)]_{k2}-CO-NR^3-[R^4-NR^3]_x-R^2-$bedeutet,

R, $R^2$, $R^3$, $R^4$, k1, k2, $Z^1$ und $Z^2$ die oben dafür angegebene Bedeutung haben,

mit der Maßgabe, dass die Summe k1+k2 $\geq$1 ist und dass mindestens ein Rest $Z^1$ oder $Z^2$ eine Hydroxy- oder $NHR^3$-Gruppe, vorzugsweise eine Hydroxygruppe, ist,

in der verbrückenden Gruppe Y damit mindestens eine Hydroxy- oder $NHR^3$-Gruppe, vorzugsweise mindestens eine Hydroxygruppe, enthalten ist.

Gegenstand der Erfindung ist weiterhin ein Verfahren zur Herstellung der wässrigen Dispersionen von vorvernetzten Organopolysiloxanen, indem

wässrige Dispersionen, vorzugsweise wässrige Emulsionen, enthaltend Aminoorganopolysiloxane (1) der Formel

$$(R^1O)_d A_e R_{3-d-e}SiO(SiARO)_p(SiR_2O)_q SiR_{3-d-e}A_e(OR^1)_d \qquad (IV),$$

wobei

A ein Aminorest der allgemeinen Formel

$$-R^2-[NR^3-R^4-]_x NR^3_2 \, ,$$

ist,

R gleich oder verschieden sein kann und einen einwertigen Kohlenwasserstoffrest mit 1 bis 18 Kohlenstoffatomen bedeutet, der ein oder mehrere Heteroatome aus der Gruppe N, P, S, O, und Halogen enthalten kann,

$R^1$ gleich oder verschieden sein kann, ein Wasserstoffatom oder einen Alkylrest mit 1 bis 18 Kohlenstoffatomen bedeutet, der durch ein oder mehrere separate Sauerstoffatome unterbrochen sein kann,

$R^2$ ein SiC-gebundener, zweiwertiger linearer oder verzweigter Kohlenwasserstoffrest mit 3 bis 18 Kohlenstoffatomen, vorzugsweise ein Alkylenrest mit 3 bis 10 Kohlenstoffatomen, bedeutet,

$R^3$ ein Wasserstoffatom, einen Alkylrest mit 1 bis 8 Kohlenstoffatomen oder einen Acylrest, wie Acetylrest, bedeutet, vorzugsweise ein Wasserstoffatom ist,

$R^4$ ein zweiwertiger Kohlenwasserstoffrest mit 1 bis 6 Kohlenstoffatomen, vorzugsweise ein Alkylenrest mit 1 bis 6 Kohlenstoffatomen, bedeutet,

d 0 oder 1 ist,

e 0 oder 1 ist,

p eine ganze Zahl von mindestens 1, vorzugsweise mindestens 2, bevorzugt mindestens 3, und höchstens 1000, vorzugsweise höchstens 10, ist und

q 0 oder eine ganze Zahl von 1 bis 2000, vorzugsweise 50 bis 1000, ist,

x 0, 1, 2, 3 oder 4, vorzugsweise 0 oder 1, ist,

mit reaktiven Estern (2) der Formel

$$R^5O_2C\text{-}[C(Z^1)(H)]_{k1}\text{-}[C(Z^2)(H)]_{k2}\text{-}CO_2R^5 \qquad (V),$$

wobei

$R^5$ gleich oder verschieden sein kann und einen O-gebundenen, gesättigten oder ungesättigten, linearen oder verzweigten, einwertigen Kohlenwasserstoffrest mit 1-20 Kohlenstoffatomen je Rest, der ein oder mehrere Heteroatome aus der Gruppe N, P, S, O und Halogen enthalten kann, bedeutet,

k1 0, 1, 2 oder 3 ist,

k2 0, 1, 2 oder 3 ist,

$Z^1$ -OH, H oder -NH$R^3$ bedeutet,

$Z^2$ -OH, H oder -NH$R^3$ bedeutet,

mit der Maßgabe, dass die Summe k1+k2 ≥1 ist und dass mindestens ein Rest $Z^1$ oder $Z^2$ eine Hydroxy- oder NH$R^3$-Gruppe, vorzugsweise eine Hydroxygruppe, ist,
umgesetzt werden.

Gegenstand der Erfindung ist weiterhin ein Verfahren zur Herstellung der vorvernetzten Organopolysiloxane, dadurch gekennzeichnet, dass

Aminoorganopolysiloxanen (1) der Formel

$$(R^1O)_dA_eR_{3\text{-}d\text{-}e}SiO(SiARO)_p(SiR_2O)_qSiR_{3\text{-}d\text{-}e}A_e(OR^1)_d \qquad (IV)$$

mit reaktiven Estern (2) der Formel

$$R^5O_2C\text{-}[C(Z^1)(H)]_{k1}\text{-}[C(Z^2)(H)]_{k2}\text{-}CO_2R^5... \qquad (V)$$

wobei

A, R, $R^1$, $R^5$, d, e, k1, k2, $Z^1$ und $Z^2$ die oben dafür angegebene Bedeutung haben,
mit der Maßgabe, dass die Summe k1+k2 ≥1 ist und dass mindestens ein Rest $Z^1$ oder $Z^2$ eine Hydroxy- oder NH$R^3$-Gruppe, vorzugsweise eine Hydroxygruppe, ist,
umgesetzt werden,
und die so erhaltenen vorvernetzten Organopolysiloxane gegebenenfalls anschließend in Wasser emulgiert werden.

Vorzugsweise werden bei dem erfindungsgemäßen Verfahren als Aminoorganopolysiloxane (1) der Formel (IV) solche eingesetzt, bei denen e gleich 0 ist, die also nur seitenständige Aminoreste A enthalten und bei der Umsetzung mit den reaktiven Estern (2) Brücken gemäß Struktureinheiten der Formel (I) bilden.

Wenn e gleich 0 ist, enthalten die erfindungsgemäßen vorvernetzten Organopolysiloxane zusätzlich zu Struktureinheiten der Formel (I) und Siloxaneinheiten der Formel (II) vorzugsweise Siloxaneinheiten der Formel

$$R_{3-d}(OR^1)_d SiO_{1/2} \qquad (III),$$

wobei R, $R^1$ und d die oben dafür angegebene Bedeutung haben.

Es können aber auch Aminoorganopolysiloxane (1) der Formel (IV) eingesetzt werden, die auch endständige Aminoreste A enthalten, bei denen e also gleich 1 ist.

Es können auch Mischungen von Aminoorganopolysiloxanen (1) der Formel (IV) mit e gleich 0 und e gleich 1 eingesetzt werden. Bei der Umsetzung mit den reaktiven Estern (2) können dabei zusätzlich Brücken zwischen zwei endständigen Aminoresten (Formel (VI)) oder zwischen endständigen und seitenständigen Aminoresten (Formel (VII)) gebildet werden.

Weiterhin können zusätzlich zu den Aminoorganopolysiloxanen (1) nur endständige Aminoreste aufweisende Aminoorganopolysiloxane (1a) der Formel

$$(R^1O)_f AR_{2-f} SiO(SiR_2O)_n SiR_{2-f} A(OR^1)_f \qquad (IVa),$$

eingesetzt werden,

wobei A, R und $R^1$ die oben dafür angegebene Bedeutung haben,

f 0 oder 1 und

n eine ganze Zahl von 1 bis 1000, vorzugsweise 50 bis 1000, ist,

mit der Maßgabe, dass durchschnittlich mindestens ein Aminorest A enthalten ist.

Die erfindungsgemäßen vorvernetzten Organopolysiloxane können daher als Brücken neben den Struktureinheiten der Formel (I) weitere Struktureinheiten der Formeln

$$SiR_2O_{1/2}\text{-}Y\text{-}SiR_2O_{1/2} \qquad (VI),$$

oder

$$SiRO_{2/2}\text{-}Y\text{-}SiR_2O_{1/2} \qquad (VII),$$

oder Mischungen aus (VI) und (VII) enthalten, wobei R und Y die oben dafür angegebene Bedeutung haben.

Bevorzugt enthalten die erfindungsgemäßen vorvernetzten Organopolysiloxane Siloxaneinheiten der Formel (III), wobei d 0 oder 1, vorzugsweise 1 ist.

Die seitenständigen und ggf. endständigen Aminoreste A in den erfindungsgemäß eingesetzten Aminoorganopolysiloxanen (1) oder ggf. mitverwendeten Aminoorganopolysiloxanen (1a) können mit den reaktiven Estern (2) gegebenenfalls auch reagieren ohne Brücken zu bilden, sodass die vorvernetzten Organopolysiloxane zusätzlich Struktureinheiten der Formeln

$$SiRO_{2/2}\text{-}R^2\text{-}[NR^3\text{-}R^4\text{-}]_x NR^3\text{-}OC\text{-}[C(Z^1)(H)]_{k1}\text{-}[C(Z^2)(H)]_{k2}\text{-}CO_2R^5 \qquad (VIIIa)$$

und/oder

$$SiR_2O_{1/2}\text{-}R^2\text{-}[NR^3\text{-}R^4\text{-}]_x NR^3\text{-}OC\text{-}[C(Z^1)(H)]_{k1}\text{-}[C(Z^2)(H)]_{k2}\text{-}CO_2R^5 \qquad (VIIIb)$$

enthalten können, wobei

R, $R^2$, $R^3$, $R^4$, $R^5$, k1, k2, x, $Z^1$ und $Z^2$ die oben dafür angegebene Bedeutung haben.

Im Rahmen dieser Erfindung soll Formel (IV) so verstanden werden, dass p Einheiten -(SiARO)- und q Einheiten -(SiR_2O)- in beliebiger Weise, beispielsweise als Block oder statistisch, im Aminorganopolysiloxanmolekül verteilt sein können.

Die erfindungsgemäß eingesetzten Aminoorganopolysiloxane (1) und ggf. mitverwendeten Aminoorganopolysiloxanen (1a) können, was durch die Formeln (IV) bzw. (IVa) nicht ausgedrückt wird, auch Siloxaneinheiten der Formeln (IXa) und/oder (IXb) und/oder (IXc)

$$RSiO_{3/2}, (OR^1)SiO_{3/2}, SiO_{4/2} \qquad (IXa\text{-}c)$$

enthalten, wobei

R und $R^1$ die oben dafür angegebene Bedeutung haben,
so dass die erfindungsgemäßen vorvernetzten Organopolysiloxane auch Siloxaneinheiten der Formeln (IXa-c) enthalten können.

Vorzugsweise ist R ein einwertiger, gesättigter oder ungesättigter Kohlenwasserstoffrest mit 1 bis 18 Kohlenstoffatomen.

Beispiele für Kohlenwasserstoffreste R sind Alkylreste, wie der Methyl-, Ethyl-, n-Propyl-, iso-Propyl-, 1-n-Butyl-, 2-n-Butyl-, iso-Butyl-, tert.-Butyl-, n-Pentyl-, iso-Pentyl-, neoPentyl-, tert.-Pentylrest; Hexylreste, wie der n-Hexylrest; Heptylreste, wie der n-Heptylrest; Octylreste, wie der n-Octylrest und iso-Octylreste, wie der 2,2,4-Trimethylpentylrest; Nonylreste, wie der n-Nonylrest; Decylreste, wie der n-Decylrest; Dodecylreste, wie der n-Dodecylrest; Octadecylreste, wie der n-Octadecylrest; Cycloalkylreste, wie der Cyclopentyl-, Cyclohexyl-, Cycloheptylrest und Methylcyclohexylreste; Alkenylreste, wie der Vinyl-, 5-Hexenyl-, Cyclohexenyl-, 1-Propenyl-, Allyl-, 3-Butenyl- und 4-Pentenylrest; Arylreste, wie der Phenyl-, Naphthyl-, Anthryl- und Phenanthrylrest; Alkarylreste, wie o-, m-, p-Tolylreste; Xylylreste und Ethylphenylreste; und Aralkylreste, wie der Benzylrest, der $\alpha$- und der $\beta$-Phenylethylrest.

Bevorzugt sind als Rest R der Methyl-, Ethyl-, Octyl- und Phenylrest, besonders bevorzugt sind der Methyl- und Ethylrest. Beispiele für substituierte Reste R sind Halogenalkylreste, wie der 3,3,3-Trifluor-n-propylrest, der 2,2,2,2' , 2' , 2'-Hexafluorisopropylrest, der Heptafluorisopropylrest und Halogenarylreste, wie der o-, m- und p-Chlorphenylrest. Weitere Beispiele für substituierte Reste R sind Polyalkylenoxygruppen, wie Polyethylenoxy-, Polypropylenoxy- oder Polyethylenoxy/Polypropylenoxy-Gruppen.

Beispiele für Rest **R**$^1$ sind die vorstehend bei **R** aufgeführten Alkylreste sowie der Methoxyethyl-, der Ethoxyethylrest und der Hexoxyethylrest, wobei es sich bei dem Rest **R**$^1$ bevorzugt um Wasserstoff und den Methyl- und den Ethylrest handelt.

Vorzugsweise ist $R^5$ ein $C_{1-20}$-Kohlenwasserstoffrest, der durch ein oder mehrere Sauerstoffatome unterbrochen ist.

Beispiele für Reste R gelten auch für Reste $R^5$.

Bevorzugt ist $R^5$ ein $C_{1-4}$-Alkylrest, wie Methyl- oder Ethylrest.

Beispiele für Reste A sind:

- $(CH_2)_3NH_2$

- $(CH_2)_3$-NH-$(CH_2)_2$-$NH_2$

-$CH_2CH(CH_3)CH_2$-NH-$(CH_2)_2$-$NH_2$

-$(CH_2)_3$-NH(Cyclohexyl)

- $(CH_2)_3$-$NHCH_3$

- $(CH_2)_3$-$N(CH_3)_2$

- $(CH_2)_3$-$NHCH_2CH_3$

- $(CH_2)_3$-$N(CH_2CH_3)_2$

- $(CH_2)_4$-$NH_2$

-$CH_2CH(CH_3)CH_2$-$NH_2$

- $(CH_2)_3$-NH-$(CH_2)_2$-$NHCH_3$

- $(CH_2)_3$-NH-$(CH_2)_2$-$N(CH_3)_2$

- $(CH_2)_3$-NH-$(CH_2)_2$-$NHCH_2CH_3$

- $(CH_2)_3$-NH-$(CH_2)_2$-$N(CH_2CH_3)_2$

- $(CH_2)_3[-NH-CH_2CH_2]_2-NH_2$

und deren teil- oder vollacetylierte Formen, wie

-$(CH_2)_3$-NH(Acetyl)

- $(CH_2)_3$-NH-$(CH_2)_2$-NH(Acetyl)

und

- $(CH_2)_3$-N(Acetyl) - $(CH_2)_2$-NH(Acetyl).

Bevorzugte Beispiele für Reste A sind:

- $(CH_2)_3NH_2$

- $(CH_2)_3$-NH-$(CH_2)_2$-NH$_2$

-$CH_2CH(CH_3)CH_2$-NH-$(CH_2)_2$-NH$_2$

- $(CH_2)_3$-NHCH$_3$

Bevorzugt ist A ein Aminorest der Formel

-$R^2$-[NH-CH$_2$CH$_2$-]$_{x'}$-NH$_2$,

wobei

x' 0 oder 1 ist und
$R^2$ ein Rest der Formel -$(CH_2)_3$- oder -$CH_2$-CH($CH_3$) -$CH_2$- ist.

Besonders bevorzugte Beispiele für Rest A sind

-$(CH_2)_3NH_2$

- $(CH_2)_3$-NH-$(CH_2)_2$-NH$_2$

und

-$CH_2CH(CH_3)CH_2$-NH-$(CH_2)_2$-NH$_2$.

Weitere Beispiele für Aminoorganopolysiloxane (1) sind handelsübliche funktionalisierte Siloxane, wie Aminöle, z.B. Aminöle mit 3-(2-Aminoethyl)aminopropylfunktionen, sowie Glykolöle, Phenyl- oder Phenylmethylöle, die Aminogruppen enthalten.

Bei der Herstellung der erfindungsgemäßen Dispersionen kann eine Art von Aminoorganopolysiloxan (1) oder verschiedene Arten von Aminoorganopolysiloxan (1) eingesetzt werden.

Die bei der Herstellung der erfindungsgemäßen Dispersionen eingesetzten Aminoorganopolysiloxane (1) weisen vorzugsweise Viskositäten von 1 mPa.s bis 50.000.000 mPa.s bei 25°C, bevorzugt 50 mPa.s bis 10.000.000 mPa.s bei 25°C und besonders bevorzugt 100 mPa.s bis 500.000 mPa.s bei 25°C auf.

Die bei der Herstellung der erfindungsgemäßen Dispersionen eingesetzten Aminoorganopolysiloxane (1) können beispielsweise, wie in US 7,129,369 B2 beschrieben, hergestellt werden.

Die erfindungsgemäßen Dispersionen enthalten vorzugsweise erfindungsgemäße vorvernetzte Organopolysiloxane, Emulgatoren (3) und
Wasser (4).

Bei der Herstellung der erfindungsgemäßen Dispersionen von vorvernetzten Organopolysiloxanen können gegebenenfalls weitere Stoffe, die an der Umsetzung nicht direkt teilnehmen, eingesetzt werden.

Die erfindungsgemäßen Dispersionen bilden beim Eintrocknen - ohne Katalysatorzugabe oder Veränderung des pH-Wertes - ein Siliconnetzwerk, vorzugsweise ein elastisches Siliconnetzwerk aus. Vorzugsweise bilden die erfindungsgemäßen Dispersionen nach dem Entfernen des Wassers elastomere Filme.

Bei dem erfindungsgemäßen Verfahren, der erfindungsgemäßen Umsetzung von Aminoorganopolysiloxanen (1) und ggf. (1a) mit reaktiven Estern (2), werden vorzugsweise keine metallhaltigen Katalysatoren mitverwendet.

Die erfindungsgemäßen Dispersionen enthalten daher vorzugsweise keine Katalysatoren.

Bei der Herstellung der erfindungsgemäßen vorvernetzten Organopolysiloxane werden Aminoorganopolysiloxane (1) und reaktive Ester (2) eingesetzt, und diese Komponenten reagieren vorzugsweise bei Raumtemperatur miteinander. Zur Unterstützung dieser Reaktion werden keine metallhaltigen, zusätzlichen Katalysatoren benötigt, d.h. es werden vorzugsweise keine Übergangsmetalle der VIII Nebengruppe des Periodensystems und deren Verbindungen und keine Metalle der III., IV. und V. Hauptgruppe des Periodensystems und deren Verbindungen verwendet, wobei die Elemente C, Si, N, und P in dieser Definition nicht als Metalle gelten.

Die Reaktion verläuft ferner vorzugsweise im neutralen Bereich, d.h. im pH-Bereich von ca. 4 bis 8, der sich durch die Komponenten selbst ergibt. Durch die hohe Reaktivität ist ferner eine gezielt geführte chemische Umsetzung nicht nötig und vorzugsweise auch kein Erwärmen.

Bei der Herstellung der erfindungsgemäßen Dispersionen kann eine Art von Ester (2) als Vernetzer oder verschiedene Arten von Estern (2) als Vernetzer eingesetzt werden.

Vorzugsweise werden als Ester (2) Tartrate der Formel

$$R^5O\text{-}OC\text{-}CH(OH)\text{-}CH(OH)\text{-}CO\text{-}OR^5$$

eingesetzt, wobei

$R^5$ die oben dafür angegebene Bedeutung hat, bevorzugt ein $C_{1-4}$-Alkylrest, insbesondere ein Methyl- oder Ethylrest ist.

Bevorzugte Beispiele für Ester (2) sind daher Diethyltartrate, wie Diethyl-L-tartrat, Diethyl-D-tartrat und meso-Diethyltartrat und Dimethyltartrate, wie Dimethyl-L-tartrat, Dimethyl-D-tartrat und meso-Dimethyltartrat wobei Diethyltartrate, wie Diethyl-L-tartrat, besonders bevorzugt sind.

Bevorzugt ist Y daher ein Rest der Formel

$$-R^2\text{-}[NH\text{-}CH_2CH_2\text{-}]_x\text{-}NH\text{-}OC\text{-}CH(OH)\text{-}CH(OH)\text{-}CO\text{-}NH\text{-}[CH_2CH_2\text{-}NH]_x\text{-}R^2\text{-}$$

wobei x 0 oder 1 ist und

$R^2$ ein Rest der Formel $-(CH_2)_3-$ oder $-CH_2\text{-}CH(CH_3)\text{-}CH_2-$ ist.

Die vorvernetzten Organopolysiloxane können je nach Einsatz von Vernetzer (2) oder linearem, verzweigtem oder harzartigem Aminoorganopolysiloxan (1) verzweigte oder sogar hochverzweigte bzw. stark vernetzte Strukturen mit linearen Anteilen haben.

Bei dem erfindungsgemäßen Verfahren werden Aminoorganopolysiloxane und Ester in Art und Menge so gewählt, dass die Organopolysiloxane in den erhaltenen Dispersionen vorvernetzt sind.

Vorzugsweise werden als Aminoorganopolysiloxane (1) solche eingesetzt, die durchschnittlich mindestens eine primäre Aminofunktion in den seitenständigen Aminoresten A enthalten, so dass erfindungsgemäße vorvernetzte Organopolysiloxane erhalten werden. Es können zusätzlich auch Aminoorganopolysiloxane (1a) eingesetzt werden, die nur endständige Aminoreste A mit primären Aminofunktionen enthalten.

Wenn Aminoorganopolysiloxane (1a) eingesetzt werden, werden sie vorzugsweise in Mengen von 10 bis 300 Gew.-teilen, je 100 Gew.-teile Aminoorganopolysiloxane (1) eingesetzt.

Ferner werden auch bei Einsatz von Gemischen aus Aminoorganopolysiloxanen (1) und unfunktionellen Organopolysiloxanen (1b), erfindungsgemäße vorvernetzte Organopolysiloxane erhalten. Unfunktionelle Organopolysiloxane (1b) sind dabei Organopolysiloxane ohne Aminoreste A, vorzugsweise lineare Organopolysiloxane der allgemeinen Formel

$$R^7_uR^6_{3-u}SiO[R^6_2SiO]_vSiR^6_{3-u}R^7_u \qquad (X)$$

wobei $R^6$ die Bedeutung von R hat und

$R^7$ die Bedeutung von $R^6$ hat oder eine HO-Gruppe ist.

u 0 oder 1 und

v 0 oder eine ganze Zahl von 1 bis 2000 ist.

Vorzugsweise ist $R^6$ ein einwertiger $C_{1-18}$-Kohlenwasserstoffrest, bevorzugt ein $C_{1-18}$-Alkylrest.

Beispiele für unfunktionelle Organopolysiloxane (1b) sind Dialkylpolysiloxane, bevorzugt Dimethylpolysiloxane.

Wenn unfunktionelle Organopolysiloxane (1b) eingesetzt werden, werden sie vorzugsweise in Mengen von 100 bis 800 Gew.-teilen je 100 Gew.-teile Aminoorganopolysiloxane (1) eingesetzt.

Der Vernetzungsgrad hängt dabei vom eingesetzten Verhältnis der Äquivalente -$OR^5$ des reaktiven Esters (2) zum Aminorest A im Aminoorganopolysiloxan (1) ab.

Zur Herstellung der erfindungsgemäßen Dispersionen aus Aminoorganopolysiloxan (1) und reaktivem Ester (2) wird der Ester (2) dabei vorzugsweise in Mengen von 0,6 bis 10 Äquivalente -$OR^5$, bevorzugt 0,65 bis 2 Äquivalente -$OR^5$, besonders bevorzugt 0,7 bis 1,5 Äquivalente -$OR^5$, je Äquivalent Aminorest A im Aminoorganopolysiloxan (1) eingesetzt,

wobei A und $R^5$ die oben dafür angegebene Bedeutung haben.

Die Herstellung der erfindungsgemäßen Dispersionen von vorvernetzten Organopolysiloxane erfolgt durch intensives Mischen von Aminoorganopolysiloxanen (1) mit Estern (2), Emulgatoren (3) und Wasser (4) miteinander. Die Herstellung kann diskontinuierlich oder kontinuierlich erfolgen.

Die Art der Mischung der Komponenten, die zur Herstellung der erfindungsgemäßen Dispersionen gebraucht werden, ist nicht sehr kritisch und kann in verschiedener Reihenfolge ausgeübt werden. Es können z.B. die Komponenten (1) und (2) miteinander vorgemischt werden, daraufhin der (oder die) Emulgator(en) zugefügt und danach Wasser (4) eingearbeitet werden. Es ist auch möglich, die Komponenten (1) bis (4) der Reihe nach in die Emulgierapparatur zu dosieren. In besonderen Fällen kann es z.B. aufgrund der Viskosität oder Reaktivität der eingesetzten Aminoorganopolysiloxane (1) und ggf. (1a) und ggf. unfunktionellen Organopolysiloxanen (1b) vorteilhaft sein, Ester (2) mit einem Aminoorganopolysiloxan zu mischen und danach ein anderes Aminoorganopolysiloxan oder unfunktionelles Organopolysiloxan einzuarbeiten, oder umgekehrt, je nachdem, wie sich günstigere rheologische Eigenschaften für die Verarbeitung der Komponenten ergeben.

Des Weiteren ist es auch möglich, den Ester (2) als Vernetzer in die fertige Emulsion von Aminoorganopolysiloxanen (1) zu geben, um so die gewünschte Reaktion und Vernetzung des Aminoorganopolysiloxans in der Emulsion zu erreichen. Um VOCfreie Produkte, also Produkte ohne flüchtige organische Verbindungen, zu erhalten kann das Nebenprodukt Alkohol $R^5OH$ (wobei $R^5$ die oben dafür angegebene Bedeutung hat) durch geeignete bekannte Maßnahmen wie Destillation, Membranverfahren oder andere Trennverfahren teilweise oder vollständig entfernt werden.

Bei der Herstellung der erfindungsgemäßen Dispersionen wird Wasser (4), in Mengen von vorzugsweise 1 bis 99 Gew.-%, besonders bevorzugt 25 bis 95 Gew.-%, jeweils bezogen auf das Gesamtgewicht aller Inhaltsstoffe der Dispersion eingesetzt.

Die erfindungsgemäße wässrige Dispersion von vorvernetzten Organopolysiloxanen wird als Öl in Wasser System eingesetzt.

Als Emulgatoren (3) zur Herstellung der wässrigen Dispersionen von vorvernetzten Organopolysiloxanen können alle bisher bekannten, anionische, nichtionische, kationische oder amphotere Emulgatoren sowohl einzeln als auch als Mischungen verschiedener Emulgatoren verwendet werden, mit denen auch bisher wässrige Dispersionen, insbesondere wässrige Emulsionen von Organopolysiloxanen hergestellt werden konnten.

Beispiele für anionische Emulgatoren sind:

1. Alkylsulfate, besonders solche mit einer Kettenlänge von 8 bis 18 C-Atomen, Alkyl- und Alkarylethersulfate mit 8 bis 18 C-Atomen im hydrophoben Rest und 1 bis 40 Ethylenoxid (EO)- bzw. Propylenoxid(PO)einheiten.

2. Sulfonate, besonders Alkylsulfonate mit 8 bis 18 C-Atomen, Alkylarylsulfonate mit 8 bis 18 C-Atomen, Tauride, Ester und Halbester der Sulfobernsteinsäure mit einwertigen Alkoholen oder Alkylphenolen mit 4 bis 15 C-Atomen; gegebenenfalls können diese Alkohole oder Alkylphenole auch mit 1 bis 40 EO-Einheiten ethoxyliert sein.

3. Alkali- und Ammoniumsalze von Carbonsäuren mit 8 bis 20 C-Atomen im Alkyl-, Aryl-, Alkaryl- oder Aralkylrest.

4. Phosphorsäureteilester und deren Alkali- und Ammoniumsalze, besonders Alkyl- und Alkarylphosphate mit 8 bis 20 C-Atomen im organischen Rest, Alkylether- bzw. Alkaryletherphosphate mit 8 bis 20 C-Atomen im Alkyl- bzw. Alkarylrest und 1 bis 40 EO-Einheiten.

Beispiele für nichtionische Emulgatoren sind:

5. Polyvinylalkohol, der noch 5 bis 50 %, vorzugsweise 8 bis 20 % Vinylacetateinheiten aufweist, mit einem Polymerisationsgrad von 500 bis 3000.

6. Alkylpolyglycolether, vorzugsweise solche mit 3 bis

40 EO-Einheiten und Alkylresten von 8 bis 20 C-Atomen.

7. Alkylarylpolyglycolether, vorzugsweise solche mit 5 bis 40 EO-Einheiten und 8 bis 20 C-Atomen in den Alkyl- und Arylresten.

8. Ethylenoxid/Propylenoxid(EO/PO)-Blockcopolymere, vorzugsweise solche mit 8 bis 40 EO- bzw. PO-Einheiten.

9. Additionsprodukte von Alkylaminen mit Alkylresten von 8 bis 22 C-Atomen mit Ethylenoxid oder Propylenoxid.

10. Fettsäuren mit 6 bis 24 C-Atomen.

11. Alkylpolyglykoside der allgemeinen Formel R" -O-$Z_O$, worin R" einen linearen oder verzweigten, gesättigten oder ungesättigten Alkylrest mit im Mittel 8 - 24 C-Atomen und $Z_O$ einen Oligoglykosidrest mit im Mittel o = 1 - 10 Hexose- oder Pentoseeinheiten oder Gemischen davon bedeuten.

12. Naturstoffe und deren Derivate, wie Lecithin, Lanolin, Saponine, Cellulose; Cellulosealkylether und Carboxyalkylcellulosen, deren Alkylgruppen jeweils bis zu 4 Kohlenstoffatome besitzen.

13. Polare Gruppen, enthaltend insbesondere die Elemente O, N, C, S, P, Si, enthaltende lineare Organo(poly)siloxane, insbesondere solche mit Alkoxygruppen mit bis zu 24 C-Atomen und/oder bis zu 40 EO- und/oder PO-Gruppen.

Beispiele für kationische Emulgatoren sind:

14. Salze von primären, sekundären und tertiären Fettaminen mit 8 bis 24 C-Atomen mit Essigsäure, Schwefelsäure, Salzsäure und Phosphorsäuren.

15. Quarternäre Alkyl- und Alkylbenzolammoniumsalze, insbesondere solche, deren Alkylgruppen 6 bis 24 C-Atome besitzen, insbesondere die Halogenide, Sulfate, Phosphate und Acetate.

16. Alkylpyridinium-, Alkylimidazolinium- und Alkyloxazoliniumsalze, insbesondere solche, deren Alkylkette bis zu 18 C-Atome besitzt, speziell die Halogenide, Sulfate, Phosphate und Acetate.

Als amphotere Emulgatoren eignen sich besonders:

17. Langkettig substituierte Aminosäuren, wie N-Alkyl-di-(aminoethyl-)glycin oder N-Alkyl-2-aminopropionsäuresalze.

18. Betaine, wie N-(3-Acylamidopropyl)-N,N-dimethylammonium-salze mit einem $C_8$-$C_{18}$-Acylrest und Alkyl-imidazolium-Betaine oder quaternisierte Alkyl oder substituierte Alkylderivate des N,N-Dimethyl-Glycins.

Bevorzugt als Emulgatoren zur Herstellung für wässrige Dispersionen vorvernetzter Organopolysiloxane sind nichtionische Emulgatoren, insbesondere die vorstehend unter 6. aufgeführten Alkylpolyglycolether.

Der Bestandteil (3) kann aus einem der o.g. Emulgatoren oder aus einem Gemisch zweier oder mehrerer o.g. Emulgatoren bestehen, er kann in reiner Form oder als Lösungen eines oder mehrerer Emulgatoren in Wasser oder organischen Lösungsmitteln eingesetzt werden.

Bei der Herstellung der erfindungsgemäßen Dispersionen werden die Emulgatoren (3) in Mengen von vorzugsweise 0,1 bis 60 Gew.-%, besonders bevorzugt 0,5 bis 30 Gew.-%, jeweils bezogen auf das Gesamtgewicht an Aminoorganopolysiloxanen (1) und Ester (2), eingesetzt.

Falls das Aminoorganopolysiloxan (1) oder der Ester (2) bzw. das entstehende vorvernetzte Organopolysiloxan selbst als Emulgator wirkt, kann auf den Zusatz von separatem Emulgator (3) verzichtet werden.

Der Emulgiervorgang zur Herstellung der Dispersion wird vorzugsweise bei Temperaturen unter 120°C, bevorzugt bei 5°C bis 100°C, besonders bevorzugt bei 10°C bis 80°C durchgeführt. Die Temperaturerhöhung kommt vorzugsweise durch den Eintrag mechanischer Scherenergie, die für den Emulgierprozess benötigt wird, zustande. Die Temperaturerhöhung wird nicht zur Beschleunigung eines chemischen Prozesses benötigt. Weiterhin erfolgt die Herstellung der Dispersionen vorzugsweise beim Druck der umgebenden Atmosphäre, sie kann aber auch bei höheren oder niederen Drücken durchgeführt werden.

Die Umsetzung von Aminoorganopolysiloxanen (1) mit Estern (2) bei der Herstellung der Dispersionen läuft vorzugsweise in wenigen Minuten bis mehreren Tagen ab.

Die bei der Herstellung der Dispersionen als Kondensationsnebenprodukte anfallenden Alkohole können im Produkt verbleiben oder auch entfernt werden, beispielsweise durch Destillation unter Vakuum, Membranverfahren, oder durch Extraktion.

Die mittels Lichtstreuung in den erfindungsgemäßen Dispersionen gemessene mittlere Teilchengröße liegt vorzugsweise im Bereich 0,001 bis 50 $\mu$m, bevorzugt bei 0,005 bis 20 um, besonders bevorzugt im Bereich 0,01 bis 10 um.

Die pH-Werte können von 1 bis 14 variieren, bevorzugt 3 bis 9, besonders bevorzugt 4 bis 8.

Gegenstand der Erfindung sind kosmetische Zusammensetzungen enthaltend erfindungsgemäße wässrige Dispersionen, vorzugsweise wässrige Emulsionen, von vorvernetzten Organopolysiloxanen.

Die erfindungsgemäße kosmetische Zusammensetzung enthält wässrige Dispersionen von vorvernetzten Organopolysiloxanen vorzugsweise in Mengen von 0,2 bis 65 Gew.-%, bevorzugt von 0,5 bis 50 Gew.-%, jeweils bezogen auf das Gesamtgewicht der kosmetischen Zusammensetzung.

Die erfindungsgemäßen kosmetischen Zusammensetzungen enthalten vorzugsweise als kosmetisch akzeptables Medium Wasser.

Die erfindungsgemäße kosmetische Zusammensetzung enthält vorzugsweise ein Konditionierungsmittel. Als Konditionierungsmittel werden analog zu K. Krummel, Stephane Chiron, J. Jachowicz, Chapter 14, in: "The Chemistry and Manufacture of Cosmetics", Volume II, Formulating, Third Edition by Mitchell L. Schlossmann, 2000, S. 359-396, kosmetische Inhaltsstoffe bezeichnet, welche die Haaroberfläche modifizieren und die Beschaffenheit des Haares beeinflussen. Kosmetische Zusammensetzungen enthaltend Konditionierungsmittel werden eingesetzt zur Modifizierung oder Verbesserung der Weichheit der Haare, besseren Entwirrbarkeit, Verringerung der Nass- und Trockenkämmkraft, Pflege der Haare, Vermeidung elektrostatischer Aufladung, leichterer Gleitwirkung durch das Haar und entlang der Haaroberfläche, Verbesserung des Haarglanzes, Erhalt der Farbechtheit von Haaren, Verringerung des Haarbruchs, Erhalt der Haarform und weiteren kosmetischen Eigenschaften, die mit natürlichem und gesundem Haar in Verbindung gebracht werden.

Die erfindungsgemäße kosmetische Zusammensetzung verbessert einen oder mehrere der oben genannten Effekte, insbesondere die Kämmbarkeit und die Deponierung des Silicons auf den Haaren.

Beispiele für Konditionierungsmittel und deren INCI-Namen sind beschrieben im "International Cosmetic Ingredient Dictionary & Handbook" des Personal Care Product Council (Hrsg.).

Als Referenz kann das World Wide Web basierte "wINCI Web Based International Cosmetic Ingredient Dictionary & Handbook (http://online.personalcarecouncil.org/jsp/Home.jsp) oder das International Cosmetic Ingredient Dictionary & Handbook, 13th Edition, The Personal Care Products Council (formerly: The Cosmetic, Toiletry, and Fragrance Association (CTFA)), 2010, Verwendung finden.

Als Konditionierungsmittel werden vorzugsweise solche ausgewählt aus der Gruppe von

kationischen Polymeren,
kationischen Tensiden,
nicht polymer vorliegenden quaternären Ammoniumverbindungen, Organopolysiloxanen und Organopolysiloxancopolymeren, die von den vorvernetzten Organopolysiloxanen enthaltend Struktureinheiten der Formel (I) verschieden sind, Fettsäureestern und Fettsäurealkoholen,
natürlichen oder synthetischen Ölen und Wachsen und Panthenol, Lipiden, Proteinen und hydrolysierten Proteinen,
sowie deren Mischungen
eingesetzt.

Bevorzugte Beispiele für Konditionierungsmittel sind kationische Polymere. Darunter verstanden werden Polymere, die seitenständig oder endständig kationische Gruppen tragen oder seitenständig oder endständig Gruppen, die durch Ionisierung in eine kationische Gruppe überführt werden können.

Vorzugsweise werden kationische Polymere verwendet, die eine quaternäre Ammoniumgruppe aufweisen.

Beispiele für vorzugsweise eingesetzte kationische Polymere sind im International Cosmetic Ingredient Dictionary & Handbook unter der Bezeichnung Polyquaternium veröffentlicht, wobei jedes Polymer mit einem individuellen Zahlenkürzel identifiziert wird, z.B. Polyquaternium-1.

Weitere Beispiele für kationische Polymere sind quaternäre Ammoniumgruppen aufweisende Derivate von modifizierten Polysacchariden, z.B. Polymere mit den INCI-Namen Cassia Hydroxypropyltrimonium Chloride, Derivate von modifizierter Cellulose und/oder Stärke, z.B. ein quaternäres Ammoniumderivat eines mit Propylenglycolether modifizierten Cyamopsis Tetragonoloba (Guar) Gums mit dem INCI-Namen Guar Hydroxypropyltrimonium Chloride, oder polymere quaternäre Ammoniumsalze des Reaktionsprodukts von Hydroxyethylcellulose mit einem Trimethylammonium-substituierten Epoxid, wie Cellulose, 2-Hydroxyethyl 2-(2-Hydroxy-3-(Trimethylammonium)Propoxy)Ethyl 2-Hydroxy-3-(Trimethylammonio)Propyl Ether Chlorid, wie Cellulose, 2-Hydroxyethyl 2-Hydroxy-3-(Trimethylammonium)Propyl Ether, Chlorid, wie Cellulose, 2-Hydroxyethyl 23-Hydroxy-3-(Trimethylammonium)Propyl Ether chloride, wie Cellulose, 2-[2-Hydroxy-3-(Trimethylammonium)Propoxy]Ethyl Ether, Chlorid, wie Cellulose, 2-[2-Hydroxy-3-Trimethylammonium)propoxy] Ethyl ether chloride mit dem INCI-Namen Polyquaternium-10.

Weitere Beispiele für kationische Polymere sind quaternäre Ammoniumgruppen aufweisende Acrylsäurepolymerderivate, Acrylsäurecopolymerderivate, Methacrylsäurederivate und Methacrylsäurecopolymerderivate, z.B. Polymere mit dem INCI-Namen Polyquaternium-37.

Weitere Beispiele für kationische Polymere sind quaternäre Ammoniumgruppen aufweisende Copolymere aus Dimethyldiallylammoniumchlorid und Acrylsäure, z.B. Polymere mit dem INCI-Namen Polyquaternium-22.

Weitere Beispiele für kationische Polymere sind quaternäre Ammoniumgruppen aufweisende Copolymere aus Derivaten des Vinylpyrrolidon, Viylimidazol und Vinylimidazolin und Methacrylsäure, z.B. Polymere mit dem INCI-Namen Polyquaternium-86.

Weitere Beispiele für kationische Polymere sind quaternäre Ammoniumgruppen aufweisende Copolymere aus Acrylamid und Dimethyl-diallyl-ammoniumchlorid, z.B. Polymere mit dem INCI-Namen Polyquaternium-7.

Weitere Beispiele für kationische Polymere sind quaternäre Ammoniumgruppen aufweisende Copolymere aus dem Reaktionsprodukt von Diethylsulfat mit Vinylpyrrolidon und Dimethylaminoethylmethacrylat, z.B. Polymere mit dem INCI-Namen Polyquaternium-11.

Wenn kationische Polymere eingesetzt werden, enthält die erfindungsgemäße kosmetische Zusammensetzung kationische Polymere vorzugsweise in Mengen von 0,01 bis 5 Gew.-%, bevorzugt von 0,05 bis 4 Gew.-%, insbesondere bevorzugt 0,10 bis 3 Gew.-%, jeweils bezogen auf das Gesamtgewicht der kosmetischen Zusammensetzung.

Weitere bevorzugte Beispiele für Konditionierungsmittel sind kationische Tenside. Beispiele für vorzugsweise eingesetzte kationische Tenside entsprechen den in Punkt 14. bis 16. unter Beispiele für kationische Emulgatoren aufgeführten Materialien.

Beispiele sind Cetyltrimethylammoniumsalze oder Behenyltrimethylammoniumsalze. Als anionisches Gegenion können beispielsweise Chlorid, Bromid, Methosulfat vorliegen. INCI-Namen von vorzugsweise eingesetzten kationischen Tensiden sind beispielsweise Cetrimonium Chloride, Cetrimonium Methosulfate, Behentrimonium Chloride, Behentrimonium Methosulfate, Steartrimonium Bromide.

Wenn kationische Tenside eingesetzt werden, enthält die erfindungsgemäße kosmetische Zusammensetzung kationische Tenside vorzugsweise in Mengen von 0,1 bis 7 Gew.-%, bevorzugt von 0,15 bis 6 Gew.-%, insbesondere bevorzugt 0,2 bis 5 Gew.-%, jeweils bezogen auf das Gesamtgewicht der kosmetischen Zusammensetzung.

Weitere Beispiele für Konditionierungsmittel sind nicht polymer vorliegende quaternäre Ammoniumverbindungen. Darunter verstanden werden nicht polymere Ammoniumverbindungen, die kationisch vorliegen oder durch Ionisierung in eine kationische Gruppe überführt werden können.

Beispiele für vorzugsweise eingesetzte nicht polymer vorliegende quaternäre Ammoniumverbindungen sind Dimethyl Dioctadecyl Ammonium Chloride mit dem INCI-Namen Distearyldimonium Chloride, N-[3-(Dimethylamino)propyl]octadecanamid mit dem INCI-Namen Stearamidopropyl Dimethylamine oder Verbindungen mit dem INCI-Namen Dicocoylethyl Hydroxyethylmonium Methosulfate oder Quaternium-87.

Weitere bevorzugte Beispiele für Konditionierungsmittel sind Organopolysiloxane und Organopolysiloxancopolymere, die von den in den wässrigen Dispersionen vorliegenden vorvernetzten Organopolysiloxanen mit Struktureinheiten der Formel (I) verschieden sind. Die Organopolysiloxane können in Form eines Öls, Wachses, Gummis oder Harzes vorliegen oder in Form einer Emulsion.

Beispiele für solche von den vorvernetzten Organopolysiloxanen mit Struktureinheiten der Formel (I) verschiedenen Organopolysiloxane sind:

Cyclische Organopolysiloxane der Formel

$$[R^*_2SiO]_x$$

wobei x eine ganze Zahl von 4 bis 8 ist,

lineare Organopolysiloxane der allgemeinen Formel

$$R^*_3SiO[R^*_2SiO]_ySiR^*_3$$

oder

$$HOSiR^*_2O[R^*_2SiO]_ySiR^*_2OH ,$$

wobei y 0 oder eine ganze Zahl von 1 bis 2000 ist,

und harzartige Organopolysiloxane der allgemeinen Formel

$$R^*_tSiO_{(4-t)/2}$$

wobei R* jeweils die oben dafür angegebene Bedeutung von R, $R^1$ oder A, vorzugsweise von R und $R^1$, hat, und t 0, 1, 2 oder 3 ist,

so dass das Organopolysiloxanharz aus M, D, T und / oder Q-Einheiten aufgebaut ist, wobei die Kombination vorwiegend oder ausschließlich aus D und T-Einheiten ebenso bevorzugt ist, wie die Kombination vorwiegend oder ausschließlich aus M und Q-Einheiten, wobei im Falle der vorwiegend oder ausschließlich aus D und T-Einheiten aufgebauten Harze T-Einheiten bevorzugt in einem Molverhältnis von T/[M+D+T+Q] von 0,45 bis 1, besonders bevorzugt von 0,55 bis 1,0 vorliegen und die Anzahl der M und Q-Einheiten in beiden Fällen vorzugsweise Null ist und in dem Falle der vorwiegend oder ausschließlich aus M und Q-Einheiten aufgebauten Organopolysiloxanharze Q-Einheiten bevorzugt in einem Molverhältnis von Q/[M+D+T+Q] von 0,25 bis 0,9, besonders bevorzugt von 0,35 bis 0,7 vorliegen und die Anzahl der D und T-Einheiten in beiden Fällen vorzugsweise Null ist.

Beispiele für Organopolysiloxane, vorliegend in Form eines Öls sind Polydimethylsiloxane mit der Viskosität 0,65 bis 2 000 000 mPas (25°C) und den INCI-Bezeichnungen Disiloxan und Dimethicone.

Weitere Beispiele für Organopolysiloxane, vorliegend in Form eines Öls oder Wachses sind funktionalisierte Organopolysiloxane, beispielsweise Polyalkylsiloxane, wobei mindestens ein Alkylrest sich unterscheidet von Methyl, beispielsweise Organopolysiloxane mit dem INCI-Namen Stearyl Dimethicone, Cetyl Dimethicone oder C26-28 Alkyl Dimethicone, oder beispielsweise Polyarylsiloxane und Polyarylalkylsiloxane, beispielsweise Organopolysiloxane mit dem INCI-Namen Phenyl Trimethicone, Trimethylsiloxyphenyl Dimethicone oder Dimethylphenyl Dimethicone, oder beispielsweise Organopolysiloxane mit einem organofunktionellen Rest wie einem Aminopropyl-, Aminopropyl-aminoethyl-, Aminopropylaminoisobutylrest, beispielsweise Organopolysiloxane mit dem INCI-Namen Amodimethicone, oder beispielsweise Organopolysiloxane mit einem Polyethylenglycol- oder Polyalkylenglycolrest, beispielsweise Organopolysiloxane mit dem INCI-Namen PEG-12 Dimethicone, PEG/PPG-25,25-Dimethicone oder Cetyl PEG/PPG-15/15 Butyl Ether Dimethicone.

Weitere Beispiele für Organopolysiloxane sind Siliconharze mit den INCI-Namen Trimethylsiloxysilicate oder Polymethylsilsesquioxane.

Wenn solche Organopolysiloxane oder Organopolysiloxancopolymere eingesetzt werden, enthält die erfindungsgemäße kosmetische Zusammensetzung Organopolysiloxane und Organopolysiloxancopolymere, die von den in den wässrigen Dispersionen vorliegenden vorvernetzten Organopolysiloxanen mit Struktureinheiten der Formel (I) verschieden sind, vorzugsweise in Mengen von 0,1 bis 40 Gew.-%, bevorzugt von 0,2 bis 30 Gew.-%, insbesondere bevorzugt 0,3 bis 20 Gew.-%, jeweils bezogen auf das Gesamtgewicht der kosmetischen Zusammensetzung.

Weitere bevorzugte Beispiele für Konditionierungsmittel sind Fettsäureester und Fettsäurealkohole.

Beispiele für Fettsäurealkohole sind Alkohole mit C8-C28 Kohlenstoffketten wie die Fettalkohole 1-Octadecanol mit dem INCI-Namen Stearyl Alcohol, 1-Hexadecanol mit dem INCI-Namen Cetyl Alcohol, oder Fettalkohole mit den INCI-Namen Cetearyl Alcohol, Myristyl Alcohol, Caprylic Alcohol, Lauryl Alcohol, Decyl Alcohol und Oleyl Alcohol.

Fettsäurealkohole erfüllen neben konditionierenden Eigenschaften auch eine strukturgebende, verdickende Wirkung bei kosmetischen Zusammensetzungen.

Weitere Beispiele für Fettsäureester sind Ester der Fettsäuren mit dem INCI-Namen Palmitic Acid, Oleic Acid, Linolic Acid, Linoleic Acid, Caprylic Acid, Myristic Acid, Stearic Acid, beispielsweise Fettsäureester mit dem INCI-Namen Isopropyl Palmitate, Ethylhexyl Palmitate, Isopropyl Myristate, Isopropyl Stearate.

Wenn Fettsäureester und Fettsäurealkohole eingesetzt werden, enthält die erfindungsgemäße kosmetische Zusammensetzung Fettsäureester und Fettsäurealkohole vorzugsweise in Mengen von 0,1 bis 15 Gew.-%, bevorzugt von 0,3 bis 12 Gew.-%, insbesondere bevorzugt 0,5 bis 10 Gew.-%, jeweils bezogen auf das Gesamtgewicht der kosmetischen Zusammensetzung.

Weitere bevorzugte Beispiele für Konditionierungsmittel sind natürliche oder synthetische Öle und Wachse.

Beispiele für bevorzugte Öle und Wachse sind Kohlenwasserstoffe mit linearen oder verzweigten, gesättigten oder ungesättigten C4-C60 Kohlenstoffketten, wie Öle und Wachse mit den INCI-Namen Isododecane, hydrated Polyisobutylene, hydrated Polydecene, Paraffin und Isoparaffin.

Weitere Beispiele für bevorzugte Öle und Wachse sind Carnaubawachs, Bienenwachs, Wollwachs, mikrokristallines Wachs, Jojobaöl, Reisöl, Calendulaöl, Sonnenblumenöl, Sojaöl, Kokosnussöl, Olivenöl und Mandelöl.

Wenn natürliche oder synthetische Öle und Wachse eingesetzt werden, enthält die erfindungsgemäße kosmetische Zusammensetzung Öle und Wachse vorzugsweise in Mengen von 0,1 bis 10 Gew.-%, bevorzugt von 0,2 bis 7 Gew.-%, insbesondere bevorzugt 0,3 bis 5 Gew.-%, jeweils bezogen auf das Gesamtgewicht der kosmetischen Zusammensetzung.

Weitere bevorzugte Beispiele für Konditionierungsmittel sind Panthenol, Lipide, wie Ceramide, Proteine und hydrolysierte Proteine, wie hydrolisiertes Kollagen, hydrolysierte Weizenproteine und hydrolysierte Seide.

Optional enthält die kosmetische Zusammensetzung weitere kosmetisch übliche Additive, wie z.B. Tenside, Verdicker, Gelierungsmittel, Filmbildner, feuchtigkeitsspendende Mittel, UV-Filter, Perlglanzpigmente, Vitamine, Antioxidantien, Coffein, Anti-Schuppenwirkstoffe oder Konservierungsmittel.

Beispiele für weitere in der Kosmetik übliche Additive und deren INCI-Namen sind beschrieben im "International Cosmetic Ingredient Dictionary & Handbook" des Personal Care Product Council.

Optional enthält die kosmetische Zusammensetzung weitere kosmetisch übliche Additive wie Tenside.

Beispiele für in der Kosmetik übliche Tenside sind auch in K. Schrader, A. Domsch, Cosmetology - Theory and Practice, Volume II, Seite II-8 bis II-22, Verlag für chemische Industrie, 2005, sowie in Punkt 1. bis 18. unter Beispiele für Emulgatoren, beschrieben.

Beispiele für vorzugsweise eingesetzte anionische Tenside entsprechen den in Punkt 1. bis 3. unter Beispiele für anionische Emulgatoren aufgeführten Materialien.

INCI-Namen von vorzugsweise eingesetzten anionische Tensiden sind beispielsweise Sodium Lauryl Sulfate, Ammonium Laureth Sulfate, Sodium Laureth Sulfate, Disodium 2-Sulfolaurate, Disodium Lauryl Sulfosuccinate oder Disodium Laureth-Sulfosuccinate.

Wenn anionische Tenside eingesetzt werden, enthält die erfindungsgemäße kosmetische Zusammensetzung anionische Tenside vorzugsweise in Mengen von 1 bis 30 Gew.-%, bevorzugt von 5 bis 25 Gew.-%, insbesondere bevorzugt 7 bis 20 Gew.-%, jeweils bezogen auf das Gesamtgewicht der kosmetischen Zusammensetzung.

Beispiele für vorzugsweise eingesetzte nichtionische Tenside entsprechen den in Punkt 5. bis 13. unter Beispiele für nichtionische Emulgatoren aufgeführten Materialien.

INCI-Namen von vorzugsweise eingesetzten nichtionischen Tensiden sind beispielsweise Coco Glucoside, Lauryl glucoside, Decyl Glucoside, PEG-40 Hydrogenated Castor Oil, Polysorbate 80 oder PEG-7 Glyceryl Cocoate.

Wenn nichtionische Tenside eingesetzt werden, enthält die erfindungsgemäße kosmetische Zusammensetzung nichtionische Tenside vorzugsweise in Mengen von 1 bis 15 Gew.-%, bevorzugt von 2 bis 12 Gew.-%, insbesondere bevorzugt 3 bis 10 Gew.-%, jeweils bezogen auf das Gesamtgewicht der kosmetischen Zusammensetzung.

Beispiele für vorzugsweise eingesetzte amphotere Tenside entsprechen den in Punkt 17. bis 18. unter Beispiele für nichtionische Emulgatoren aufgeführten Materialien. Weitere bevorzugte Beispiele sind Verbindungen aus den Klassen der Alkylamidobetaine, Alykylamphoacetate und Alkylamphopropionate. INCI-Namen von vorzugsweise eingesetzten nichtionischen Tensiden sind beispielsweise Cocamidopropyl Betaine, Cetyl Betaine, Cocamide MEA, Cocamide DEA, Cocamide MIPA, Sodium Cocoamphoacetate und Sodium Cocoamphopropionate.

Wenn amphotere Tenside eingesetzt werden, enthält die erfindungsgemäße kosmetische Zusammensetzung amphotere Tenside vorzugsweise in Mengen von 1 bis 15 Gew.-%, bevorzugt von 2 bis 12 Gew.-%, insbesondere bevorzugt 3 bis 10 Gew.-%, jeweils bezogen auf das Gesamtgewicht der kosmetischen Zusammensetzung.

Optional enthält die kosmetische Zusammensetzung weitere kosmetisch übliche Additive wie Verdicker.

Beispiele für vorzugsweise eingesetzte Verdicker sind modifizierte Polysaccharide wie Stärke, Cellulose, Gummi Arabicum und Guar Gums, z.B. Polymere mit dem INCI-Namen Cellulose Gum, Guar Gum, Xanthan Gum oder Cassia Gum.

Weitere Beispiele für Verdicker sind hydrophob modifizierte nichtionische Cellulosederivate, z.B. das Cellulosederivat mit dem INCI-Namen Hydroxyethylcellulose.

Weitere Beispiele für Verdicker sind vernetzte Acrylsäure- und Methacrylsäurepolymere und Derivate der vernetzten Acrylsäure- und Methacrylsäurepolymere, z.B. Polymere mit dem INCI-Namen Carbomer.

Weitere Beispiele für Verdicker sind Agentien, die in Kombination mit Tensiden eine verdickende Wirkung erzielen. Beispiele sind Monoglyceride von Fettsäuren, Mono/Diglyceride von ethoxylierten Fettsäuren und ethoxylierte Fettalkohole. INCI-Namen von vorzugsweise eingesetzten Verdickern die in Kombination mit Tensiden eine verdickende Wirkung erzielen sind PEG-120 Methyl Glucose Dioleate, PEG-150 Distearate, Myristyl Glycol, PEG-200 Glyceryl Palmitate, Laureth-4 oder PEG-200 Glyceryl Palmitate.

Weitere Beispiele für Verdicker sind Salze, z.B. Salze mit dem INCI-Namen Sodium Chloride.

Wenn Verdicker eingesetzt werden, enthält die erfindungsgemäße kosmetische Zusammensetzung Verdicker vorzugsweise in Mengen von 0,1 bis 10 Gew.-%, jeweils bezogen auf das Gesamtgewicht der kosmetischen Zusammensetzung.

Optional enthält die kosmetische Zusammensetzung weitere kosmetisch übliche Additive wie Filmbildner.

Bevorzugte Beispiele für Filmbildner sind Polymere.

Beispiele für vorzugsweise eingesetzte filmbildende Polymere sind beschrieben im "International Cosmetic Ingredient Dictionary & Handbook" des Personal Care Product Councils.

Beispiele für bevorzugte filmbildende Polymere sind Acrylsäurepolymerderivate, Acrylsäurecopolymerderivate, Methacrylsäurederivate und Methacrylsäurecopolymerderivate.

Beispiele für bevorzugte anionische Polymere sind Copolymere aus Vinyl Acetate und einem oder mehrerer Acrylsäure-, Methacrylsäuremonomere und deren Ester, z.B. Polymere mit dem INCI-Namen Acrylates/VA Copolymer.

Weitere Beispiele für bevorzugte filmbildende Polymere sind Copolymere aus Vinylpyrrolidone und einem oder mehrerer Acrylsäure-, Methacrylsäuremonomere und deren Ester, z.B. Polymere mit dem INCI-Namen Acrylates/VP Copolymer.

Weitere Beispiele für bevorzugte filmbildende Polymere sind Copolymere aus tert-Butyl Acrylamide und einem oder mehrerer Acrylsäure-, Methacrylsäuremonomere und deren Ester, z.B. Polymere mit dem INCI-Namen Acrylates/t-Butylacrylamide Copolymer.

Weitere Beispiele für bevorzugte filmbildende Polymere sind Copolymere aus Vinyl Acetate, Crotonsäure und Vinyl Neodecanoate Monomeren, z.B. Polymere mit dem INIC-Namen VA/Crotonates/Vinyl Neodecanoate Copolymer.

Weitere Beispiele für bevorzugte filmbildende Polymere sind Copolymere aus Vinyl Acetate, Crotonsäure und Vinyl Neodecanoate Monomeren und Vinylsiliconen, z.B. Polymere mit dem INCI-Namen Crotonic Acid/Vinyl C8-C12 Isoalkyl Esters/VA/Bis-Vinyldimethicone Copolymer.

Wenn filmbildende Polymere eingesetzt werden, enthält die erfindungsgemäße kosmetische Zusammensetzung filmbildende Polymere vorzugsweise in Mengen von 0,1 bis 15 Gew.-%, bevorzugt von 0,2 bis 10 Gew.-%, insbesondere bevorzugt 0,3 bis 7 Gew.-%, jeweils bezogen auf das Gesamtgewicht der kosmetischen Zusammensetzung.

Optional enthält die kosmetische Zusammensetzung weitere kosmetisch übliche Additive wie feuchtigkeitsspendende Mittel.

Beispiele für vorzugsweise eingesetzte feuchtigkeitsspendende Mittel sind Glycerin, Sorbitol, Xylitol, Polyethylenglycol, 1,2-Propandiol, 1,3-Propandiol oder Polypropylenglykol.

Wenn feuchtigkeitsspendende Mittel eingesetzt werden, enthält die erfindungsgemäße kosmetische Zusammensetzung feuchtigkeitsspendende Mittel vorzugsweise in Mengen von 0,1 bis 10 Gew.-%, bevorzugt von 0,2 bis 8 Gew.-%, insbesondere bevorzugt 0,3 bis 6 Gew.-%, jeweils bezogen auf das Gesamtgewicht der kosmetischen Zusammensetzung.

Optional enthält die kosmetische Zusammensetzung weitere kosmetisch übliche Additive wie perlglanzgebende Mittel.

Beispiele für vorzugsweise eingesetzte perlglanzgebende Mittel sind Perlglanzpigmente oder Glycol Distearate.

Wenn Perlglanz gebende Mittel eingesetzt werden, enthält die erfindungsgemäße kosmetische Zusammensetzung Perlglanz gebende Mittel vorzugsweise in Mengen von 0,1 bis 7 Gew.-%, bevorzugt von 0,2 bis 6 Gew.-%, insbesondere bevorzugt 0,3 bis 5 Gew.-%, jeweils bezogen auf das Gesamtgewicht der kosmetischen Zusammensetzung.

Die kosmetischen Zusammensetzungen werden vorzugsweise hergestellt durch Mischen mindestens einer erfindungsgemäßen wässrigen Dispersion von vorvernetzten Organopolysiloxanen mit gegebenenfalls mindestens einem Konditionierungsmittel und ggf. weiteren kosmetisch üblichen Additiven in einem kosmetisch akzeptablen Medium, bevorzugt Wasser.

Die einzelnen Inhaltsstoffe können in einem heiß/heiß-, heiß/kalt- oder kalt/kalt-Verfahren miteinander vermischt werden.

Die Zugabe der erfindungsgemäßen Dispersionen von vorvernetzten Organopolysiloxanen beim Herstellen der erfindungsgemäßen kosmetischen Zusammensetzung erfolgt vorzugsweise bei Temperaturen von höchstens 50°C, bevorzugt bei Temperaturen von höchstens 40°C, besonders bevorzugt bei Temperaturen von höchstens 35°C. Sie erfolgt vorzugsweise bei Temperaturen von mindestens 5°C, bevorzugt bei Temperaturen von mindestens 10°C.

Die erfindungsgemäße kosmetische Zusammensetzung kann vorliegen in Form einer Emulsion, einer Suspension, einer Lösung, einer Creme, einer Lotion, eines Schaums, eines Stifts, eines Seifenstücks, einer Paste, eines Gels.

Die erfindungsgemäße kosmetische Zusammensetzung in Form einer Emulsion kann vorliegen in Form einer W/O-Emulsion (Wasser-in-Öl-Emulsion), einer O/W-Emulsion (Öl-in-Wasser-Emulsion) oder als multiple Emulsion.

Ist das Ziel, eine kosmetische Zusammensetzung enthaltend erfindungsgemäße wässrige Dispersion von vorvernetzten Organopolysiloxanen in Form einer Emulsion in transluzenter oder transparenter Erscheinungsform herzustellen, werden bevorzugt erfindungsgemäße wässrige Dispersionen von vorvernetzten Organopolysiloxanen mit Teilchengrößen < 700 nm eingesetzt, besonders bevorzugt mit Teilchengrößen < 400 nm, insbesondere bevorzugt mit Teilchengrößen < 300 nm.

Ein weiterer Gegenstand der Erfindung ist die Verwendung der erfindungsgemäßen kosmetischen Zusammensetzung zur Behandlung keratinischer Fasern, wie Haaren. Vorzugsweise werden die kosmetischen Zusammensetzungen zur Reinigung und Pflege von keratinischen Fasern, wie Haaren, verwendet.

Beispiele für Mittel zur Reinigung und Pflege von Haaren sind Haar-Shampoos, -Spülungen (Rinse-Off Conditioner), -Kuren, - Masken, -Seren, -Schäume, -Stylingsprays, -Cremes, -Gele, -Öle, -Spitzenfluids und -Färbemittel.

Ein weiterer Gegenstand der Erfindung ist die Verwendung der erfindungsgemäßen kosmetischen Zusammensetzung zum Konditionieren von keratinischen Fasern, wie Haaren, um insbesondere die Kämmbarkeit zu erleichtern.

Ein weiterer Gegenstand der Erfindung ist ein Verfahren zur Behandlung von keratinischen Fasern, vorzugsweise Haaren, indem die erfindungsgemäßen kosmetischen Zusammensetzungen auf die keratinischen Fasern, vorzugsweise Haare, aufgetragen werden und dann gegebenenfalls mit Wasser gespült werden.

In den nachstehend beschriebenen Beispielen beziehen sich alle Angaben von Teilen und Prozentsätzen, falls nicht anders angegeben, auf das Gewicht. Des Weiteren beziehen sich alle Viskositätsangaben auf eine Temperatur von 25°C. Sofern nicht anders angegeben, werden die nachstehenden Beispiele bei einem Druck der umgebenden Atmosphäre, also etwa 1020 hPa, und bei Raumtemperatur, also bei etwa 20°C, bzw. bei einer Temperatur, die sich beim Zusammengeben der Reaktanten bei Raumtemperatur ohne zusätzliche Heizung oder Kühlung einstellt, durchgeführt.

**Herstellung der Aminosiliconöl-Emulsionen E1 - E7:**

**Herstellung der Aminosiliconöl-Emulsion E1:**

Mit einem Ultra-Turrax T 50 Emulgiergerät (Fa. Janke & Kunkel / IKA) werden bei 4000 U/min 4,9 g einer 80%-igen wässrigen Lösung von Isotridecyldecaethoxylat, käuflich erwerblich unter dem Handelsnamen Lutensol TO 10 (Fa. BASF), und 1,4 g vollentsalztes Wasser vorgemischt. 34,8 g eines hydroxy/methoxyterminierten Copolymers aus 3-(2-Aminoethylamino)propyl-methylsiloxy- und Dimethylsiloxy-einheiten mit einer Aminzahl von 0,13 und einer Viskosität von 4000 $mm^2$/s (bei 25°C) werden in drei Portionen bei einer Scherung von 4000 U/min zugegeben, so dass eine relativ feste steife Phase als Voremulsion resultiert. Es wird mit 57,8 g vollentsalztem Wasser portionsweise unter geringer Scherung zur gewünschten Emulsion verdünnt und mit 0,20 g 80%-iger Essigsäure und mit 0,9 g 2-Phenoxyethanol versetzt.

Man erhält eine glatte, niederviskose, weiße Silikonöl-Emulsion mit einem Festgehalt von 40% und einem pH-Wert von 5.

Die Teilchengrößenverteilung ist monomodal mit einem D50 Wert von 110 nm. Die Emulsion ist auch nach 4-wöchiger Lagerung bei 50°C homogen und stabil.

**Herstellung der Aminosiliconöl-Emulsion E2:**

Mit einem Ultra-Turrax T 50 Emulgiergerät (Fa. Janke & Kunkel / IKA) werden bei 4000 U/min 5,5 g einer 80%-igen wässrigen Lösung von Isotridecyldecaethoxylat, käuflich erwerblich unter dem Handelsnamen Lutensol TO 10 (Fa. BASF), und 5,5 g vollentsalztes Wasser vorgemischt. 34,8 g eines hydroxy/methoxyterminierten Copolymers aus 3-(2-Aminoethylamino)propyl-methylsiloxy- und Dimethylsiloxy-einheiten mit einer Aminzahl von 0,25 und einer Viskosität von 4000 $mm^2$/s (bei 25°C) werden in drei Portionen bei einer Scherung von 4000 U/min zugegeben, so dass eine relativ feste steife Phase als Voremulsion resultiert. Es wird mit 53,0 g vollentsalztem Wasser portionsweise unter geringer Scherung zur gewünschten Emulsion verdünnt und mit 0,25 g 80%-iger Essigsäure und mit 0,9 g 2-Phenoxyethanol versetzt.

Man erhält eine glatte, niederviskose, weiße Silikonöl-Emulsion mit einem Festgehalt von 40% und einem pH-Wert von 6,5.

Die Teilchengrößenverteilung ist bimodal mit einem D50 Wert von 110 nm. Die Emulsion ist auch nach 4-wöchiger Lagerung bei 50°C homogen und stabil.

**Herstellung der Aminosiliconöl-Emulsion E3:**

Mit einem Ultra-Turrax T 50 Emulgiergerät (Fa. Janke & Kunkel / IKA) werden bei 4000 U/min eine Mischung aus 1,5 g Isotridecylpentaethoxylat, käuflich erwerblich unter dem Handelsnamen Lutensol TO 5 (Fa. BASF), 3,0 g Isotridecyloctaethoxylat, käuflich erwerblich unter dem Handelsnamen Lutensol TO 8, und 5,0 g vollentsalztes Wasser vorgemischt. 26,1 g eines trimethylsilyl-terminierten Copolymers aus 3-(2-Aminoethyl-amino)propyl-methylsiloxy- und Dimethylsiloxy-Einheiten mit einer Aminzahl von 0,6 und einer Viskosität von 1000 $mm^2$/s (bei 25°C) werden in drei Portionen bei einer Scherung von 4000 U/min zugegeben, so dass eine relativ feste steife Phase als Voremulsion resultiert. Es wird mit 62,7 g vollentsalztem Wasser portionsweise unter geringer Scherung zur gewünschten Emulsion verdünnt und mit 0,75 g 80%-iger Essigsäure und mit 0,9 g 2-Phenoxyethanol versetzt.

Man erhält eine glatte, niederviskose, partiell durchsichtige bläuliche Silikonöl-Emulsion mit einem Festgehalt von 31,5% und einem pH Wert von 6,5.

Die Teilchengrößenverteilung ist monomodal mit einem D50 Wert von 56 nm. Die Emulsion ist auch nach 4-wöchiger Lagerung bei 50°C homogen und stabil.

**Herstellung der Aminosiliconöl-Emulsion E4:**

Mit einem Ultra-Turrax T 50 Emulgiergerät (Fa. Janke & Kunkel / IKA) werden bei 4000 U/min 5,5 g einer 80%-igen wässrigen Lösung von Isotridecyldecaethoxylat, käuflich erwerblich unter dem Handelsnamen Lutensol TO 10 (Fa. BASF), und 5,5 g vollentsalztes Wasser vorgemischt. Eine Mischung aus 12,6 g eines hydroxy/methoxy-terminierten Copolymers aus 3-(2-Aminoethyl-amino)propyl-methylsiloxy- und Dimethylsiloxy-einheiten mit einer Aminzahl von 0,13 und einer Viskosität von 4000 $mm^2$/s (bei 25°C) und 22,2 g eines trimethylsilyl-terminierten Polydimethylsiloxans mit einer Viskosität von 60000 mm$^2$/s (bei 25°C) werden in drei Portionen bei einer Scherung von 4000 U/min zugegeben, so dass eine relativ feste steife Phase als Voremulsion resultiert. Es wird mit 53,2 g vollentsalztem Wasser portionsweise unter geringer Scherung zur gewünschten Emulsion verdünnt und mit 0,08 g 80%-iger Essigsäure und mit 0,9 g 2-Phenoxyethanol versetzt.

Man erhält eine glatte, niederviskose, weiße Silikonöl-Emulsion mit einem Festgehalt von 40% und einem pH-Wert von 5.

Die Teilchengrößenverteilung ist bimodal mit einem D50 Wert von 219 nm. Die Emulsion ist auch nach 4-wöchiger Lagerung bei 50°C homogen und stabil.

**Herstellung der Aminosiliconöl-Emulsion E5:**

Mit einem Ultra-Turrax T 50 Emulgiergerät (Fa. Janke & Kunkel / IKA) werden bei 4000 U/min 5,5 g einer 80%-igen wässrigen Lösung von Isotridecyldecaethoxylat, käuflich erwerblich unter dem Handelsnamen Lutensol TO 10 (Fa. BASF), und 5,5 g vollentsalztes Wasser vorgemischt. Eine Mischung aus 10,2 g eines trimethylsilyl-terminierten Copolymers aus 3-(2-Amino-ethylamino)propyl-methylsiloxy- und Dimethylsiloxy-einheiten mit einer Aminzahl von 0,6 und einer Viskosität von 2100 mm$^2$/s (bei 25°C) und 24,6 g eines trimethylsilyl-terminierten Polydimethylsiloxans mit einer Viskosität von 60000 mm$^2$/s (bei 25°C) werden in drei Portionen bei einer Scherung von 6000 U/min zugegeben, so dass eine relativ feste steife Phase als Voremulsion resultiert. Es wird mit 53,0 g vollentsalztem Wasser portionsweise unter geringer Scherung zur gewünschten Emulsion verdünnt und mit 0,30 g 80%-iger Essigsäure und mit 0,9 g 2-Phenoxyethanol versetzt.

Man erhält eine glatte, niederviskose, weiße Silikonöl-Emulsion mit einem Festgehalt von 40% und einem pH Wert von 5.

Die Teilchengrößenverteilung ist monomodal mit einem D50 Wert von 186 nm. Die Emulsion ist auch nach 4-wöchiger Lagerung bei 50°C homogen und stabil.

**Herstellung der Aminosiliconöl-Emulsion E6:**

Mit einem Ultra-Turrax T 50 Emulgiergerät (Fa. Janke & Kunkel / IKA) werden bei 4000 U/min 5,5 g einer 80%-igen wässrigen Lösung von Isotridecyldecaethoxylat, käuflich erwerblich unter dem Handelsnamen Lutensol TO 10 (Fa. BASF), und 5,5 g vollentsalztes Wasser vorgemischt. Eine Mischung aus 17,1 g eines trimethylsilyl-terminierten Copolymers aus 3-(2-Aminoethylamino)propyl-methylsiloxy- und Dimethylsiloxy-einheiten mit einer Aminzahl von 0,6 und einer Viskosität von 2100 mm$^2$/s (bei 25°C) und 17,7 g eines trimethylsilyl-terminierten Polydimethylsiloxans mit einer Viskosität von 60000 mm$^2$/s (bei 25°C) werden in drei Portionen bei einer Scherung von 6000 U/min zugegeben, so dass eine relativ feste steife Phase als Voremulsion resultiert. Es wird mit 52,8 g vollentsalztem Wasser portionsweise unter geringer Scherung zur gewünschten Emulsion verdünnt und mit 0,49 g 80%-iger Essigsäure und mit 0,9 g 2-Phenoxyethanol versetzt.

Man erhält eine glatte, mittelviskose, weiße Silikonöl-Emulsion mit einem Festgehalt von 40% und einem pH Wert von 5.

Die Teilchengrößenverteilung ist multimodal mit einem D50 Wert von 171 nm. Die Emulsion ist auch nach 4-wöchiger Lagerung bei 50°C homogen und stabil.

**Herstellung der Aminosiliconöl-Emulsion E7:**

Mit einem Ultra-Turrax T 50 Emulgiergerät (Fa. Janke & Kunkel / IKA) werden bei 4000 U/min 5,5 g einer 80%-igen wässrigen Lösung von Isotridecyldecaethoxylat, käuflich erwerblich unter dem Handelsnamen Lutensol TO 10 (Fa. BASF), und 5,5 g vollentsalztes Wasser vorgemischt. Eine Mischung aus 17,4 g eines Copolymers aus hydroxy-/methoxyterminierten 3-(2-Amino-ethylamino)propyl-methylsiloxy- und Dimethylsiloxy-einheiten mit einer Aminzahl von 0,13 und einer Viskosität von 4000 mm$^2$/s (bei 25°C) und 17,4 g eines (3-Aminopropyl)-dimethylsilyl-terminierten Polydimethylsiloxans mit einer Aminzahl von 0,04 und einer Viskosität von 13.100 mPas (bei 25°C) werden in drei Portionen bei einer Scherung von 6000 U/min zugegeben, so dass eine relativ feste steife Phase als Voremulsion resultiert. Es wird mit 53,2 g vollentsalztem Wasser portionsweise unter geringer Scherung zur gewünschten Emulsion verdünnt und mit 0,23 g 80%-iger Essigsäure und mit 0,9 g 2-Phenoxyethanol versetzt.

Man erhält eine glatte, niederviskose, weiße Silikonöl-Emulsion mit einem Festgehalt von 40% und einem pH Wert von 5.

Die Teilchengrößenverteilung ist bimodal mit einem D50 Wert von 115 nm. Die Emulsion ist auch nach 4-wöchiger Lagerung bei 50°C homogen und stabil.

**Beispiele 1-6:**

Die nachfolgenden Beispiele 1-6 repräsentieren Herstellungsverfahren zur Synthese von erfindungsgemäßen wässrigen Dispersionen von vorvernetzten Organopolysiloxanen, die zur Herstellung von erfindungsgemäßen kosmetischen Zusammensetzungen eingesetzt werden.

**Beispiel 1: Emulsion B1**

Mit einem Ultra-Turrax T 50 Emulgiergerät (Fa. Janke & Kunkel / IKA) werden bei 5000 U/min 98,9 g der Aminosiliconöl-Emulsion **E1** mit 1,1 g Diethyl-L-Tartrat innerhalb einer Minute homogenisiert.

Man erhält eine glatte, niederviskose, weiße Silikonöl-Emulsion **B1** mit einem Festgehalt von 41% und einem pH-Wert von 5. Die Teilchengrößenverteilung ist monomodal mit einem D50 Wert von 110 nm. Die Emulsion ist auch nach 4-wöchiger Lagerung bei 50°C homogen und stabil.

Durch Eindampfen der Emulsion wird nach einer Trocknungszeit von 24 Stunden bei 25°C ein weiß-opaker, gelartiger, auf Glas und Aluminium schwach haftender, auf der Oberfläche klebriger Film erhalten.

**Beispiel 2: Emulsion B2**

Mit einem Ultra-Turrax T 50 Emulgiergerät (Fa. Janke & Kunkel / IKA) werden bei 5000 U/min 98,9 g der Aminosiliconöl-Emulsion **E2** mit 1,1 g Diethyl-L-Tartrat innerhalb einer Minute homogenisiert.

Man erhält eine glatte, niederviskose, weiße Silikonöl-Emulsion **B2** mit einem Festgehalt von 41% und einem pH-Wert von 6,5. Die Teilchengrößenverteilung ist bimodal mit einem D50 Wert von 110 nm. Die Emulsion ist auch nach 4-wöchiger Lagerung bei 50°C homogen und stabil.

Durch Eindampfen der Emulsion wird nach einer Trocknungszeit von 24 Stunden bei 25°C ein weiß-opaker, elastischer, auf Glas und Aluminium gut haftender, auf der Oberfläche nicht klebriger Film erhalten.

**Beispiel 3: Emulsion B3**

Mit einem Ultra-Turrax T 50 Emulgiergerät (Fa. Janke & Kunkel / IKA) werden bei 5000 U/min 98,9 g der Aminosiliconöl-Emulsion **E3** mit 1,1 g Diethyl-L-Tartrat innerhalb einer Minute homogenisiert.

Man erhält eine glatte, niederviskose, weiße Silikonöl-Emulsion **B3** mit einem Festgehalt von 41% und einem pH-Wert von 6,5. Die Teilchengrößenverteilung ist bimodal mit einem D50 Wert von 110 nm. Die Emulsion ist auch nach 4-wöchiger Lagerung bei 50°C homogen und stabil.

Durch Eindampfen der Emulsion wird nach einer Trocknungszeit von 24 Stunden bei 25°C ein weiß-opaker, elastischer, auf Glas und Aluminium gut haftender, auf der Oberfläche nicht klebriger Film erhalten.

**Beispiel 4: Emulsion B4**

Mit einem Ultra-Turrax T 50 Emulgiergerät (Fa. Janke & Kunkel / IKA) werden bei 5000 U/min 98,9 g der Aminosiliconöl-Emulsion **E4** mit 1,1 g Diethyl-L-Tartrat innerhalb einer Minute homogenisiert.

Man erhält eine glatte, niederviskose, transluzente Emulsion **B4** mit einem Festgehalt von 33% und einem pH-Wert von 6,5. Die Teilchengrößenverteilung ist monomodal mit einem D50 Wert von 56 nm. Die Emulsion ist auch nach 4-wöchiger Lagerung bei 50°C homogen und stabil.

Durch Eindampfen der Emulsion wird nach einer Trocknungszeit von 24 Stunden bei 25°C ein weiß-opaker, elastischer, auf Glas und Aluminium gut haftender, auf der Oberfläche nicht klebriger Film erhalten.

**Beispiel 5: Emulsion B5**

Mit einem Ultra-Turrax T 50 Emulgiergerät (Fa. Janke & Kunkel / IKA) werden bei 5000 U/min 98,9 g einer Emulsion **E5** mit 1,1 g Diethyl-L-Tartrat innerhalb einer Minute homogenisiert.

Man erhält eine glatte, niederviskose, weiße Silikonöl-Emulsion **B5** mit einem Festgehalt von 41% und einem pH-Wert von 5. Die Teilchengrößenverteilung ist monomodal mit einem D50 Wert von 186 nm. Die Emulsion ist auch nach 4-wöchiger Lagerung bei 50°C homogen und stabil.

Durch Eindampfen der Emulsion wird nach einer Trocknungszeit von 24 Stunden bei 25°C ein weiß-opaker, gelartiger, auf Glas und Aluminium schwach haftender, auf der Oberfläche klebriger Film erhalten.

**Beispiel 6: Emulsion B6**

Mit einem Ultra-Turrax T 50 Emulgiergerät (Fa. Janke & Kunkel/IKA) werden bei 5000 U/min 98,9 g einer Emulsion **E6** mit 1,1 g Diethyl-L-Tartrat innerhalb einer Minute homogenisiert. Man erhält eine glatte, mittelviskose, weiße Silikonöl-Emulsion **B6** mit einem Festgehalt von 41% und einem pH-Wert von 5. Die Teilchengrößenverteilung ist multimodal mit einem D50 Wert von 171 nm. Die Emulsion ist auch nach 4-wöchiger Lagerung bei 50°C homogen und stabil.

Durch Eindampfen der Emulsion wird nach einer Trocknungszeit von 24 Stunden bei 25°C ein weiß-opaker, gelartiger, auf Glas und Aluminium schwach haftender, auf der Oberfläche klebriger Film erhalten.

**Beispiel 7: Emulsion B7**

Mit einem Ultra-Turrax T 50 Emulgiergerät (Fa. Janke & Kunkel / IKA) werden bei 5000 U/min 98,9 g einer Emulsion **E7** mit 1,1 g Diethyl-L-Tartrat innerhalb einer Minute homogenisiert.

Man erhält eine glatte, niederviskose, weiße Silikonöl-Emulsion **B7** mit einem Festgehalt von 41% und einem pH-Wert von 5. Die Teilchengrößenverteilung ist bimodal mit einem D50 Wert von 115 nm. Die Emulsion ist auch nach 4-wöchiger Lagerung bei 50°C homogen und stabil.

Durch Eindampfen der Emulsion wird nach einer Trocknungszeit von 24 Stunden bei 25°C ein weiß-opaker, gelartiger, auf Glas und Aluminium schwach haftender, auf der Oberfläche klebriger Film erhalten.

**Beispiel 8:**

Mit einem Ultra-Turrax T 50 Emulgiergerät (Fa. Janke & Kunkel / IKA) werden bei 4000 U/min 4,9 g einer 80%-igen wässrigen Lösung von Isotridecyldecaethoxylat, käuflich erwerblich unter dem Handelsnamen Lutensol TO 10 (Fa. BASF), und 2,4 g vollentsalztes Wasser vorgemischt. 34,4 g eines hydroxy/methoxy-terminierten Copolymers aus 3-(2-Aminoethyl-amino)propyl-methylsiloxy- und Dimethylsiloxy-einheiten mit einer Aminzahl von 0,13 und einer Viskosität von 4000 mm$^2$/s (bei 25°C) werden in drei Portionen bei einer Scherung von 4000 U/min zugegeben, so dass eine relativ feste steife Phase resultiert. Diese steife Phase wird mit 4,9 g vollentsalztem Wasser weiter vorverdünnt. Jetzt arbeitet man 1,1 g Diethyl-L-Tartrat weiter bei 4000 U/min ein.

Es wird mit 51,2 g vollentsalztem Wasser portionsweise unter geringer Scherung zur gewünschten Emulsion verdünnt und mit 0,20 g 80%-iger Essigsäure und mit 0,9 g 2-Phenoxyethanol versetzt.

Man erhält eine glatte, niederviskose, weiße Silikonöl-Emulsion **B8** mit einem Festgehalt von 40% und einem pH-Wert von 5.

Die Teilchengrößenverteilung ist monomodal mit einem D50 Wert von 105 nm. Die Emulsion ist auch nach 4-wöchiger Lagerung bei 50°C homogen und stabil.

Durch Eindampfen der Emulsion wird nach einer Trocknungszeit von 24 Stunden bei 25 °C ein weiß-opaker, gelartiger, auf Glas und Aluminium schwach haftender, auf der Oberfläche klebriger Film erhalten.

**Beispiel 9:**

Mit einem Ultra-Turrax T 50 Emulgiergerät (Fa. Janke & Kunkel / IKA) werden bei 4000 U/min 5,5 g einer 80%-igen wässrigen Lösung von Isotridecyldecaethoxylat, käuflich erwerblich unter dem Handelsnamen Lutensol TO 10 (Fa. BASF), und 5,5 g vollentsalztes Wasser vorgemischt. Eine Mischung aus 33,76 g eines Copolymers aus 3-(2-Aminoethylamino)propyl-methylsiloxy- und Dimethylsiloxy-einheiten mit einer Aminzahl von 0,13 und einer Viskosität von 4000 mm$^2$/s (bei 25°C) und 1,04 g Diethyl-L-Tartrat werden in drei Portionen bei einer Scherung von 4000 U/min zugegeben, so dass eine relativ feste steife Phase als Voremulsion resultiert. Es wird mit 53,1 g vollentsalztem Wasser portionsweise unter geringer Scherung zur gewünschten Emulsion verdünnt und mit 0,20 g 80%-iger Essigsäure und mit 0,9 g 2-Phenoxyethanol versetzt.

Man erhält eine glatte, niederviskose, weiße Silikonöl-Emulsion **B9** mit einem Festgehalt von 40% und einem pH Wert von 5.

Die Teilchengrößenverteilung ist bimodal mit einem D50 Wert von 134 nm. Die Emulsion ist auch nach 4-wöchiger Lagerung bei 50°C homogen und stabil.

Durch Eindampfen der Emulsion wird nach einer Trocknungszeit von 24 Stunden bei 25 °C ein weiß-opaker, gelartiger, auf Glas und Aluminium schwach haftender, auf der Oberfläche klebriger Film erhalten.

**Beispiel 10:**

Eine Mischung aus 97,0 g eines hydroxy/methoxy-terminierten Copolymers aus 3-(2-Aminoethyl-amino)propyl-methylsiloxy- und Dimethylsiloxy-einheiten mit einer Aminzahl von 0,13 und einer Viskosität von 4000 mm$^2$/s (bei 25°C) und 3,0 g Diethyl-L-Tartrat werden mit einem Flügelrührer an einem Rührgerät der Firma IKA gut homogenisiert. Anschließend wird sofort ein Film gegossen, wie unten beschrieben. Man erhält einen transparenten Elastomerfilm.

**Vergleichsversuche V1 und V2:**

Die nachfolgenden Vergleichsversuche V-1 und V-2 repräsentieren Herstellverfahren zur Synthese wässriger nichterfindungsgemäßer Emulsionen

**Vergleichsversuch V1** (Vernetzung mit nicht-erfindungsgemäßem Bernsteinsäure-diethylester):

Mit einem Ultra-Turrax T 50 Emulgiergerät (Fa. Janke & Kunkel / IKA) werden bei 5000 U/min 99,1 g der Aminosiliconöl-Emulsion E1 mit 0,9 g Bernsteinsäure-diethylester innerhalb einer Minute homogenisiert.
Man erhält eine glatte, niederviskose, weiße Silikonöl-Emulsion **V1** mit einem Festgehalt von 40% und einem pH-Wert von 5. Die Teilchengrößenverteilung ist monomodal mit einem D50 Wert von 110 nm. Die Emulsion ist auch nach 4-wöchiger Lagerung bei 50°C homogen und stabil.
Durch Eindampfen der Emulsion wird nach einer Trocknungszeit von 24 Stunden bei 25 °C eine weiß-opake, auf Glas und Aluminium schwach haftende, pastöse Schicht erhalten.

**Vergleichsversuch V2** (Vernetzung mit nicht-erfindungsgemäßem Diethyloxalat gemäß US 5,039,738 A):

Mit einem Ultra-Turrax T 50 Emulgiergerät (Fa. Janke & Kunkel / IKA) werden bei 5000 U/min 99,1 g der Aminosiliconöl-Emulsion E1 mit 0,9 g Diethyloxalat innerhalb einer Minute homogenisiert.
Man erhält eine glatte, niederviskose, weiße Silikonöl-Emulsion **V2** mit einem Festgehalt von 40% und einem pH Wert von 5. Die Teilchengrößenverteilung ist monomodal mit einem D50 Wert von 110 nm. Die Emulsion ist auch nach 4-wöchiger Lagerung bei 50°C homogen und stabil.
Durch Eindampfen der Emulsion wird nach einer Trocknungszeit von 24 Stunden bei 25 °C eine weiß-opake, auf Glas und Aluminium schwach haftende, pastöse Schicht erhalten.

**Rheologie der Elastomerfilme nach Entfernen von Wasser:**

*Herstellung der Filme für Rheologiemessungen:*

Auf eine Glas-Rundplatte mit 65 mm Durchmesser wird ein Teflonring mit 40 mm Innendurchmesser gelegt, um so eine Gussform zu bilden. In diesen Ring werden 2,5 g der zu vermessenden Emulsion auf die Glasplatte blasenfrei eingewogen. Anschließend lässt man die gegossene Emulsion bei 25°C und 101,425 kPa trocknen. Es ist darauf zu achten, dass sich die Gussform mit der gegossenen Emulsion zum Trocknen auf einer nivellierten ebenen Fläche befindet, damit sich ein gleichmäßig dicker Film ausbilden kann. Man erhält hier nach der Trocknung einen ca. 0,5 mm dicken Film.
Nach 1 Woche Standzeit bei 25 °C werden die erhaltenen Filme rheologisch untersucht. Die Messungen erfolgten mit einem Rheometer "MCR 302" der Firma Anton Paar, wobei ein Platte-Platte-Messsystem PP12.5 bei einer Spalthöhe von 0,5 mm verwendet wurde. Die Kalibrierung des Gerätes erfolgte mit Normalöl 10000 der Physikalisch-Technischen Bundesanstalt. Die Messtemperatur beträgt 25,00°C +/- 0,05°C.
Die in Tabelle 1 aufgeführten Werte des Speichermoduls G', Verlustmoduls G", und des Verlustfaktors tan $\delta$ können errechnet werden, indem durch Vorgabe einer sinusförmigen Deformation die Schubspannung z und der Phasenverschiebungswinkel $\delta$ gemessen werden. Die in Tabelle 1 aufgeführten Messwerte wurden bei einer Frequenz von 1 Hz und bei einer Deformation von 0.1% gemessen. Bei einer Deformation liegen die gemessenen Proben im linear visko-elastischen Messbereich. Dabei gilt: tan $\delta$ = G"/G'. Wenn tan $\delta$ < 1 überwiegt der elastische Charakter der Probe, wenn tan $\delta$ > 1 überwiegt der viskose Charakter der Probe.
Die Ergebnisse der Rheologiemessungen für die Elastomerfilme der erfindungsgemäßen Beispiele B1 bis B10, der Vergleichsversuche V-1 und V2 sowie der unvernetzten Aminosiliconöl-Emulsionen E1 bis E7 sind in Tabelle 1 zusammengefasst.

**Tabelle 1:** Rheologische Daten der Elastomerfilme

| Beisp./Vergl. | Speichermodul G' [Pa] | Verlustmodul G'' [Pa] | tan $\delta$ |
|---|---|---|---|
| B1 | 20600 | 5930 | 0,29 |

(fortgesetzt)

| Beisp./Vergl. | Speichermodul G' [Pa] | Verlustmodul G'' [Pa] | tan $\delta$ |
|---|---|---|---|
| B2 | 10600 | 2630 | 0,25 |
| B3 | 37900 | 7890 | 0,21 |
| B4 | 2060 | 1290 | 0,63 |
| B5 | 15600 | 1880 | 0,12 |
| B6 | 29100 | 5080 | 0,18 |
| B7 | 9020 | 4010 | 0,45 |
| B8 | 13200 | 4190 | 0,32 |
| B9 | 13200 | 4190 | 0,32 |
| B10 | 53200 | 16300 | 0,31 |
| V-1 | 231 | 725 | 3,13 |
| V-2 | 11900 | 7860 | 0,66 |
| E1 | 53 | 319 | 6,05 |
| E2 | 405 | 723 | 1,78 |
| E3 | 18,1 | 248 | 13,6 |
| E4 | 12 | 211 | 18,1 |
| E5 | 1,3 | 19,6 | 15,3 |
| E6 | 100 | 451 | 4,5 |
| E7 | 143 | 765 | 5,4 |

Tabelle 1 zeigt, dass die erfindungsgemäßen Emulsionen B1 bis B9 mit Diethyl-L-tartrat als Vernetzerkomponente nach Entfernen von Wasser elastische Filme bilden, da tan $\delta$ < 1. Auch beim Beispiel 10 wird ein elastischer Film gebildet.

Die nicht-erfindungsgemäße Emulsion V2 (enthält Diethyloxalat als Vernetzer) bildet nach Entfernen von Wasser ebenfalls einen elastischen Film.

Im Gegensatz dazu bildet die nicht-erfindungsgemäße Emulsion V1, welche Bernsteinsäurediethylester anstatt Diethyl-L-tartrat enthält, keinen Film. Dies zeigt der Verlustfaktor von 3,13.

Die eingedampften, reinen Aminosiliconöl-Emulsionen E1 bis E7 zeigen nach dem Entfernen des Wassers ebenfalls keine Filmbildung, da tan $\delta$ in allen Fällen größer 1 ist. Dies zeigt, dass die Zugabe eines Vernetzers erforderlich ist um eine Filmbildung zu erreichen.

Überraschenderweise zeigen diese Ergebnisse, dass durch Zugabe des erfindungsgemäßen, hydroxyfunktionellen Vernetzers Diethyl-L-Tartrat die Filmbildung erfolgt, nicht aber durch Zugabe des nicht-erfindungsgemäßen, unfunktionalisierten Diesters, wie Bernsteinsäurediethylester.

### Testmethoden zur Beurteilung der Wirkung von kosmetischen Zusammensetzungen:

*Naturhaar*

Die Beurteilung des Applikationsverhaltens der kosmetischen Zusammensetzung und deren Wirkung bezüglich Kämmkraft und Weichheit erfolgte auf kaukasischem Haar, erhältlich bei der Firma Kerling International Haarfabrik GmbH. Ungeschädigte Naturhaartressen werden vor ihrem Einsatz gereinigt und in einem weiteren Prozessschritt eventuell durch Bleichen geschädigt.

*Grundreinigung*

Zur Reinigung werden die ungeschädigten Haartressen eine Stunde in ein Lösungsmittelgemisch aus gleichen Teilen Toluol und Isobutylketon eingelegt und geschüttelt. Nach Entfernen des Lösungsmittelgemischs werden die Haartressen jeweils zweimal mit 3 ml einer Ammonium Lauryl Sulfat Lösung (25%ig), STEPANOL(R) ALS 25, Fa. STEPAN Company und anschließend 30°C warmen, voll entsalztem Wasser gewaschen. Dabei werden die Tressen mit einem grobzinkigen Kamm entwirrt. Anschließend werden die Haartressen eine Stunde in einem großen Becherglas in vollentsalztes Wasser gelegt, entnommen, und nochmals unter fließendem, vollentsalztem Wasser gespült. Nach der Grundreinigung werden die Tressen vor ihrer Weiterverwendung mindestens 12 Stunden bei 23°C und 50% Luftfeuchte konditioniert und vor ihrer Verwendung gekämmt.

*Bleichen von Haaren - Erzeugung geschädigter Haare*

Geschädigte Haare werden durch Bleichen gereinigter Naturhaartressen erzeugt. Hierzu werden jeweils fünf Haartressen für 30 Minuten in eine Lösung aus 30%igem Wasserstoffperoxid und 25%igem Ammoniak (Verhältnis 33,5:1) eingelegt. Anschließend werden die Haare sorgfältig mit voll entsalztem Wasser ausgespült und zweimal mit 3 ml einer Ammonium Lauryl Sulfat Lösung (25%ig), STEPANOL(R) ALS 25, Fa. STEPAN Company und 30°C warmen, voll entsalztem Wasser gewaschen. Danach werden die Haartressen eine Stunde in einem großen Becherglas in vollentsalztes Wasser gelegt, entnommen, und nochmals unter fließendem, vollentsalzten Wasser gespült. Vor einer weiteren Behandlung werden die gebleichten Tressen mindestens 12 Stunden bei 23°C und 50% Luftfeuchte konditioniert und vor ihrer Verwendung gekämmt.

*Kämmkraftmessung:*

Zur Bestimmung der Kämmkraft von nassem und trockenem Haar wurden Haartressen aus geschädigtem, kaukasischem Haar der Fa. Kerling International Haarfabrik GmbH (Haartressen Schädigungsgrad B, doppelt gezogen) mit einem Gewicht von 2 g und einer Länge von 20 cm verwendet. Die Messung der Kämmkraft mit Doppelkammmethode nach Y. K. Kamath und Hans-Dietrich Weigmann, J. Soc. Cosmet. Chem., 37, 111-124, 1986 erfolgte mit einer Zug-Dehnungsmaschine Instron 3343. Zunächst wird die Nass- und Trockenkämmkraft entlang der Messstrecke an unbehandelten Haartressen bestimmt. Anschließend werden die Haartressen mit einer erfindungsgemäßen kosmetischen Zusammensetzung behandelt und die Kraftaufnahme beim Kämmvorgang bestimmt. Als Messwert wird die Reduktion der Kämmkraft entlang der Messstrecke (Arbeit) angegeben, die sich zwischen der behandelten und unbehandelten Haartresse ergibt. Es wird der Mittelwert aus fünf Haartressen gebildet. Die Angabe der Kämmkraftreduktion erfolgt in Prozent.

*Geschmeidigkeit/Weichheit (gemäß Zug-Prüfung) :*

Zur Bestimmung der Weichheit des Haars wurden Haartressen Fa. Kerling International Haarfabrik GmbH (kaukasisches Haar, doppelt gezogen) mit einem Gewicht von 2 g und einer Länge von 20 cm verwendet. Die Bestimmung der Haarweichheit im trockenen Zustand erfolgte durch Einsatz einer Zug-Prüfmaschine Instron 3343, indem die benötigte Zugkraft mit den Parametern Biegesteifigkeit und Oberflächenrauigkeit des Haarbündels in Korrelation gesetzt wird. Diese beiden Parameter wiederum korrelieren mit der Haarweichheit. Zu diesem Zweck wurde eine unbehandelte Haartresse in eine Messanordnung eingespannt, die aus fünf versetzt gegenüberliegenden Stäben besteht. Die Form der Haartresse in dieser Ausgangsposition ist eine Art Doppel-S. Die Haartresse wird nach dieser Vorbereitung in einer Richtung aus der Messanordnung gezogen und die notwendige Kraft entlang der Messstrecke als Arbeit ausgewertet. Anschließend werden die Haartressen mit einer erfindungsgemäßen kosmetischen Zusammensetzung behandelt und die Kraftaufnahme beim Ziehen der Haartresse durch die Messanordnung entlang der Messstrecke bestimmt. Als Messwert wird die Reduktion der Zugkraft entlang der Messstrecke (Arbeit) angegeben, die sich zwischen der behandelten und unbehandelten Haartresse ergibt. Eine hohe Reduktion der Zugkraft (Arbeit) entspricht einem guten Weichgriff bzw. einer hohen Geschmeidigkeit. Es wird der Mittelwert aus fünf Haartressen gebildet.

*Weichheit (gemäß Panel-Test) :*

Zur Beurteilung der Weichheit von Haartressen werden dessen haptische Eigenschaften durch Experten (trainierte Panelisten) beurteilt. Es erfolgt jeweils ein paarweiser Vergleich von Haartressen, z.B. der Vergleich von Shampoo-behandelten Haaren zu unbehandelten Haaren. Die Zahl der beurteilten Tressenpaare beträgt mindestens drei, die Zahl der Panelisten mindestens fünf. Die Evaluierung basierte auf Haartressen der Fa. Kerling International Haarfabrik GmbH (kaukasisches Haar, doppelt gezogen) mit einem Gewicht von 2 g und einer Länge von 20 cm.

*Aufwaschprozedur Shampoo:*

Auf eine gereinigte, befeuchtete Haartresse wird 0,2 g Shampoo pro g Haar appliziert. Das Shampoo wird 30 Sekunden lang in Richtung der Haarspitzen einmassiert. Anschließend wird die Haartresse 30 s unter fließendem, voll entsalztem Wasser ausgespült und mit einem grobzinkigen Kamm entwirrt. Die Prozedur wird zweimal wiederholt. Beim letzten Mal wird der Spülprozess auf 60 s verlängert. Anschließend wird die Haartresse mindestens 12 h bei einer Luftfeuchte von 50% und einer Temperatur von 23°C getrocknet.

*Aufwaschprozedur Conditioner:*

Auf eine gereinigte, befeuchtete Haartresse wird 0,3 g Rinse-Off Conditioner / g Haar appliziert. Der Rinse-Off Conditioner wird 120 Sekunden lang in Richtung der Haarspitzen einmassiert. Anschließend wird die Haartresse 60 s unter fließendem, voll entsalztem Wasser ausgespült und mit einem grobzinkigen Kamm entwirrt. Die Prozedur wird wiederholt. Anschließend wird die Haartresse mindestens 12 h bei einer Luftfeuchte von 50% und einer Temperatur von 23°C getrocknet.

*Bestimmung der abgeschiedenen Menge Si auf der Haaroberfläche in ppm (Silicondeponierung):*

Zur Bestimmung der abgeschiedenen Menge an Silicon auf der Haaroberfläche wird ein energiedispersives Röntgenfluoreszenz-Spektrometer (AMETEK, XEPOS) genutzt. Die Haarbündel werden in einem speziell angefertigten Probenhalter platziert, der eine kreisrunde Messfläche von 12 mm Durchmesser aufweist. Die Haaroberfläche im Bereich der Messfläche ist glatt, die Haare sind parallel ausgerichtet. Die Probe wird unter Heliumatmosphäre durch Einsatz einer Palladiumröhre angeregt (17,05 kV, 2,0 mA). Die Anregungsdauer beträgt 300 s. Kontrollproben (Naturhaartressen) werden regelmäßig gemessen. Bei Abweichungen erfolgt eine Driftkorrektur mit Glastabletten. Als Kalibrierstandards wurden Haartressen verwendet, die mit Polydimethylsiloxan im Bereich 50 bis 2000 ppm beladen waren (Kontrolle durch Atomabsorptionsspektroskopie).

Zur Bestimmung der Effektivität der Siliconabscheidung wird zunächst die Menge Si in ppm eines gereinigten Haarbündels bestimmt = Blindwert. Anschließend wird das gleiche Haarbündel z.B. durch Waschen mit einem Shampoo behandelt. Die Menge an Si in ppm wird erneut bestimmt = Probenwert. Die abgeschiedene Menge an Si in ppm ergibt sich durch Durchführung der Subtraktion Probenwert - Blindwert. Jede Haartresse wird mittig auf der Vorderseite und Rückseite vermessen. Als Ergebnis wird der Mittelwert aus drei Haartressen angegeben.

*Simulation von Shampoowäschen durch Rühren von Haartressen in einer Tensidlösung:*

Viele Nutzer wünschen nach Anwendung eines Haarkonditionierenden Produktes eine Persistenz kosmetischer Effekte wie verbesserte Haarweichheit, Nasskämmkraftreduktion, Erhalt der Haarfarbe trotz mehrerer darauf folgender Wäschen mit Shampoo. Zur Beurteilung der Persistenz kosmetischer Effekte nach Behandlung von Haaren mit einer erfindungsgemäßen kosmetischen Zusammensetzung wurde eine Methode entwickelt, die eine Simulation von aufeinanderfolgenden Shampoowäschen darstellt. Dazu wird eine behandelte Haartresse in einem 100 ml Schraubdeckelglas mit 50 ml einer auf 40°C temperierten, fünfprozentigen Lösung von Ammonium Lauryl Sulfat, erhalten durch Verdünnen von STEPANOL® ALS 25, Fa. STEPAN Company, versetzt und eine vorgegebene Zeit in einem auf 40°C temperierten Inkubationsschüttler (Fa Heidolph Unimax 1010 + Incubator 1000) mit einer Geschwindigkeit von 250 rpm geschüttelt. Nach dem Schütteln werden die Tressen eine Minute mit 30°C warmen, vollentsalztem Wasser gespült und getrocknet.

*Farbschutz / Farbmessung:*

Eine Beurteilung der erfindungsgemäßen kosmetischen Zusammensetzung bezüglich der Farbschutzwirkung auf keratinischen Fasern erfolgte auf gefärbtem Echthaar. Hierzu wurden Haartressen mit einem Gewicht von 4 g und einer Länge von 20 cm aus geschädigtem, kaukasischem Haar der Fa. Kerling International Haarfabrik GmbH verwendet (Klebetresse dicht aus Euro-Haar, Bleichstufe A, Mischung 79). Die Haartressen wurden rot gefärbt. Als Farbton wurde KOLESTON PERFECT® 77/44 der Firma WELLA oder Majirel MIX ROUGE der Firma L'Oreal eingesetzt. 50 ml Haarfarbpaste wurden mit 80 ml einer sechsprozentigen Wasserstoffperoxidlösung vermischt. Die Paste wurde gleichmäßig auf die Haartressen (1,6 g Paste/g Haar) aufgetragen. Nach 40 Minuten Einwirkzeit bei Raumtemperatur wurde die Farbpaste aus den Haartressen ausgewaschen. Nach Behandlung der Haartresse mit einer Tensidlösung (auf 5% Aktivgehalt verdünntes STEPANOL® ALS 25, Fa. STEPAN Company) und Trocknung der Tressen wurde der Färbevorgang im Fall des Einsatzes von KOLESTON PERFECT® 77/44 wiederholt.

Die Farbmessung erfolgt an der glatten Oberfläche der Haarbündel mit dem Farbmessgerät Spectro Guide der Firma Byk-Gardner. Die Farbparameter L, a, b (Lab-Farbraum) werden protokolliert.

*Beurteilung des Farbschutzes durch eine Haarbehandlung mit erfindungsgemäßen kosmetischen Zusammensetzungen:*

Gefärbte Haare verändern den Farbeindruck nach Waschen der Haare mit Shampoo. Die Änderung des Farbeindrucks kann man durch den $\Delta$E-Wert beschreiben, der definiert ist als:

$$\Delta E = ((L1-Lo)^2 + (a1-a0)^2 + (b1-b0)^2)^{1/2}$$

L0, a0, b0 sind die Farbwerte eines gefärbten, unbehandelten Haarbündels.

L1, a1 und b1 sind die Farbwerte des Haarbündels nach Simulation von Shampoowäschen durch Rühren von Haartressen in einer Tensidlösung. Ein kleiner $\Delta$E-Wert ist Hinweis für eine kleinere Farbänderung bzw. erhöhte Persistenz.

**Beispiele für kosmetische Zusammensetzungen:**

**Beispiele A1 bis A4** (Rinse-Off Conditioner (Spülung) **A1 - A4**:

Die nachfolgenden Beispiele repräsentieren erfindungsgemäße kosmetische Zusammensetzungen **A1** - **A4** gemäß Tabelle 2 enthaltend die Emulsionen **B1** und **B2** aus den Beispielen 1 und 2. Der Aktivgehalt an vorvernetztem Organopolysiloxan in der kosmetischen Zusammensetzung beträgt 0,5 bis 2 %.

Herstellanweisung:

Wasser wird vorgelegt und unter Rühren auf 75°C erwärmt. Dabei werden 1,3 Teile Hydroxyethylcellulose zugegeben. Wenn 65°C erreicht sind, werden 0,5 Teile Polysorbate 80, 0,5 Teile Stearyl Alcohol, 0,5 Teil Cetyl Alcohol und 0,2 Teile Behentrimonium Chloride zugegeben. Die Mischung wird gerührt bis 75°C erreicht sind und die Inhaltsstoffe gelöst vorliegen. Dann wird die Mischung abgekühlt. Während des Abkühlens werden 0,1 Teile Citric Acid und 0,2 Teile Tetrasodium EDTA zugegeben. Bei 35°C werden 0,9 Teile Phenoxyethanol, Ethylhexylglycerin zugegeben. Unter weiterem Rühren erfolgt die Zugabe der Emulsion aus den Beispielen. Die Zusammensetzung wird 15 Minuten unter Rühren homogenisiert.

**Tabelle 2:** Rinse-Off Conditioner **A1 - A4**

| Bestandteile (INCI-name) | Bsp. **A1** [Gew.-teile] | Bsp. **A2** [Gew.-teile] | Bsp. **A3** [Gew.-teile] | Bsp. **A4** [Gew.-teile] |
|---|---|---|---|---|
| Water | ad 100 | ad 100 | ad 100 | ad 100 |
| Hydroxyethylcellulose[1] | 1,3 | 1,3 | 1,3 | 1,3 |
| Cetyl Alcohol [2] | 0,5 | 0,5 | 0, 5 | 0,5 |
| Polysorbate 80 [3] | 0,5 | 0,5 | 0,5 | 0,5 |
| Behentrimonium Chloride [4] | 0,2 | 0,2 | 0,2 | 0,2 |
| Stearyl Alcohol [5] | 0,5 | 0,5 | 0,5 | 0,5 |
| Citric Acid [6] | 0,1 | 0,1 | 0,1 | 0,1 |
| Tetrasodium EDTA [7] | 0,2 | 0,2 | 0,2 | 0,2 |
| Emulsion **B1** aus Bsp. 1 | 5,71 | | | |
| Emulsion **B1** aus Bsp. 1 | | 1,43 | | |
| Emulsion **B2** aus Bsp. 2 | | | 5,71 | |
| Emulsion **B2** aus Bsp. 2 | | | | 1,43 |
| Phenoxyethanol, Ethylhexylglycerin [8] | 0,9 | 0,9 | 0,9 | 0,9 |

Die in Tabelle 2 genannten Rohstoffe sind unter folgenden Handelsnamen erhältlich:

1)Hydroxyethylcellulose: Natrosol 250 HR, Ashland
2)Cetylalkohol: Cetylalkohol, Merck KGaA
3) Polysorbate 80: Tween™ 80, Croda GmbH
4) Behentrimonium Chloride: Genamin® KDMP, Clariant GmbH
5) Stearylalkohol: Stearylalkohol Merck KGaA
6)Citric Acid: Citric Acid, Sigma
7)Tetrasodium EDTA: EDETA® B Pulver, BASF Corporation
8)Phenoxyethanol, Ethylhexylglycerin: Euxyl PE 9010, Schülke & Mayr

**Vergleichsversuche V-A1 bis V-A5** (Rinse-Off-Conditioner (Spülung) V-A1 - V-A5, nicht erfindungsgemäß)

Die nachfolgenden Vergleichsversuche **V-A1** bis **V-A5** repräsentieren nicht-erfindungsgemäße kosmetische Zusammensetzungen **V-A1** - **V-A4** enthaltend eine wässrige Dispersion eines nichtvorvernetzten Organopolysiloxans bzw. eine nicht-erfindungsgemäße kosmetische Zusammensetzungen enthaltend eine vorvernetzte wässrige Dispersion unter Verwendung des nicht-erfindungsgemäßen Oxalsäurediethylesters. Der Aktivgehalt an Organopolysiloxan beträgt 0,5 bis 2 Gew-%. Bei der Herstellung der kosmetischen Zusammensetzungen der Vergleichsversuche **V-A1** bis **V-A5** wurde die Arbeitsweise der Beispiele **A1-A4** wiederholt, mit der Abänderung, dass anstelle der Emulsionen **B1** und **B2** aus Beispielen 1 und 2 (Emulsionen von erfindungsgemäßen vorvernetzten Organopolysiloxanen) die unvernetzten Emulsionen **E1** und **E2** bzw. die Emulsionen des nicht erfindungsgemäßen Vergleichsversuchs **V2** eingesetzt werden. Emulsionen der Vergleichsversuche **E1** und **E2** enthalten zu den Beispielen B1 und B2 bezüglich Ausgangsviskosität analoge, aber unvernetzte Organopolysiloxane. Die Emulsionen des Vergleichsversuchs **V2** ist mit dem nicht erfindungsgemäßen Diethyloxalat anstelle des erfindungsgemäßen Diethyl-L-tartrat vorvernetzt.

**Tabelle 3:** Rinse-Off Conditioner **V-A1** - **VA5**

| Bestandteile (INCI-name) | Vgl.- versuch **V-A1** [Gew.-teile] | Vgl.- versuch **V-A2** [Gew.-teile] | Vgl.- versuch **V-A3** [Gew.-teile] | Vgl.- versuch **V-A4** [Gew.-teile] | Vgl.- versuch **V-A5** [Gew.-teile] |
|---|---|---|---|---|---|
| Water | ad 100 | ad 100 | ad 100 | ad 100 | ad 100 |
| Hydroxyethyl-cellulose [1] | 1,3 | 1,3 | 1,3 | 1,3 | 1,3 |
| Cetyl Alcohol [2] | 0,5 | 0,5 | 0,5 | 0,5 | 0,5 |
| Polysorbate 80 [3] | 0,5 | 0,5 | 0,5 | 0,5 | 0,5 |
| Behentrimonium Chloride [4] | 0,2 | 0,2 | 0,2 | 0,2 | 0,2 |
| Stearyl Alcohol [5] | 0,5 | 0,5 | 0,5 | 0,5 | 0,5 |
| Citric Acid [6] | 0,1 | 0,1 | 0,1 | 0,1 | 0,1 |
| Tetrasodium EDTA [7] | 0,2 | 0,2 | 0,2 | 0,2 | 0,2 |
| Emulsion **E1** aus Bsp. 1 | 5,71 | | | | |
| Emulsion **E1** aus Bsp. 1 | | 1,43 | | | |
| Emulsion **E2** aus Bsp. 2 | | | 5,71 | | |
| Emulsion **E2** aus Bsp. 2 | | | | 1,43 | |
| Emulsion **V2** aus Vergl.-versuch V2 | | | | | 5,71 |
| Phenoxyethanol, Ethylhexylglycerin [8] | 0,9 | 0,9 | 0,9 | 0,9 | 0,9 |

Die in Tabelle 3 genannten Rohstoffe sind unter folgenden Handelsnamen erhältlich:

1)Hydroxyethylcellulose: Natrosol 250 HR, Ashland
2)Cetylalkohol: Cetylalkohol, Merck KGaA
3) Polysorbate 80: Tween™ 80, Croda GmbH
4) Behentrimonium Chloride: Genamin® KDMP, Clariant GmbH
5) Stearylalkohol: Stearylalkohol Merck KGaA
6)Citric Acid: Citric Acid, Sigma
7) Tetrasodium EDTA: EDETA® B Pulver, BASF Corporation
8)Phenoxyethanol, Ethylhexylglycerin: Euxyl PE 9010, Schülke & Mayr

Herstellanweisung:

Wasser wird vorgelegt und unter Rühren auf 75°C erwärmt. Dabei werden 1,3 Teile Hydroxyethylcellulose zugegeben. Wenn 65°C erreicht sind, werden 0,5 Teile Polysorbate 80, 0,5 Teile Stearyl Alcohol, 0,5 Teil Cetyl Alcohol und 0,2 Teile Behentrimonium Chloride zugegeben. Die Mischung wird gerührt bis 75°C erreicht sind und die Inhaltsstoffe gelöst vorliegen. Dann wird die Mischung abgekühlt. Während des Abkühlens werden 0,1 Teile Citric Acid und 0,2 Teile Tetrasodium EDTA zugegeben. Bei 35°C werden 0,9 Teile Phenoxyethanol, Ethylhexylglycerin zugegeben. Unter weiterem Rühren erfolgt die Zugabe der Emulsion gemäß Tabelle 3 für die Vergleichsversuche. Die Zusammensetzung wird 15 Minuten unter Rühren homogenisiert.

**Vergleich der Rinse-Off Conditioner der erfindungsgemäßen Beispiele A1-A4 mit den Vergleichsversuchen V-A1 bis V-A4**

Die angeführten Beispiele und Vergleichsversuche unterscheiden sich dadurch, dass im Fall der Beispiele **A1-A4** erfindungsgemäße wässrige Dispersionen von vorvernetzten Organopolysiloxanen Einsatz fanden, in Vergleichsversuch **V-A1** bis **V-A4** jeweils analoge, wässrige Dispersionen der entsprechenden nicht vorvernetzten Organopolysiloxane. Der Aktivgehalt an Organopolysiloxan in der kosmetischen Zusammensetzung beträgt 0,5 Gew-%.

Im direkten Vergleich korrelieren folgende Beispiele / Vergleichsversuche:

Beispiel A1 - Vergleichsversuch V-A1
Beispiel A2 - Vergleichsversuch V-A2
Beispiel A3 - Vergleichsversuch V-A3
Beispiel A4 - Vergleichsversuch V-A4

**Nasskämmkraft nach Behandlung von geschädigtem Haar mit Rinse-Off Conditioner und nach Simulierung mehrerer Shampoowäschen durch Rühren der Haare in einer wässrigen Tensidlösung (Persistenzeffekt)**

Beispiel **A2** - Vergleichsversuch **V-A2**

Der Einsatz der wässrigen Dispersion **B1** von erfindungsgemäß vorvernetzten Organopolysiloxanen im Rinse-Off Conditioner (Beispiel **A2**) führt zu einer Verbesserung von konditionierenden Eigenschaften wie z.B. der Verringerung der Kämmkräfte im nassen Zustand im Vergleich zum nicht erfindungsgemäßen Rinse-Off Conditioner aus Beispiel **V-A2**, der das nicht vorvernetzte Organopolysiloxan **E1** enthält. Ziel der erfindungsgemäßen Rinse-Off Conditioner ist zusätzlich, dass die pflegenden Eigenschaften auch nach mehreren Shampoowäschen der Haare erhalten bleiben. Der Prozess der Shampoowäsche wird in diesem Beispiel durch das vierstündige Rühren von Rinse-Off-Conditioner behandelten Haarbündeln in einer Tensidlösung simuliert. Die Details dieser Behandlung sind oben unter *Testmethoden* beschrieben.

Die Ergebnisse der Bestimmung der Kämmkraft im nassen Zustand sind nachfolgend für die Rinse-Off Conditioner der Beispiele **A2** und **V-A2** in Tabelle 4 aufgeführt.

**Tabelle 4:** Rinse-Off Conditioner / Ergebnisse der Nasskämmkraftreduktion auf geschädigtem kaukasischem Haar nach Behandlung mit einem erfindungsgemäßen Rinse-Off Conditioner im Vergleich zu einem nicht-erfindungsgemäßen Conditioner und nach Simulierung mehrerer Shampoowäschen durch vierstündiges Rühren der Haare in einer wässrigen Tensidlösung. Alle Ergebnisse beziehen sich auf den Vergleich zu unbehandelten Haartressen.

| Bsp. / Vgl. | Reduktion Nasskämmkraft nach Conditionerbehandlung [%] | Reduktion Nasskämmkraft nach Conditionerbehandlung und anschließendem Rühren der Haare in einer wässrigen Tensidlösung (Persistenztest) [%] |
|---|---|---|
| A2 | 89 | 47 |
| V-A2 | 85 | 34 |

Durch Behandlung mit dem erfindungsgemäßen Rinse-Off Conditioner enthaltend eine wässrige Emulsion eines vorvernetzten Organopolysiloxans **B1** (Beispiel **A2**) kann mit 89% eine signifikante Reduktion der Nasskämmkraft der Haartressen gemessen werden. Insbesondere bleibt eine sehr hohe konditionierende Wirkung nach Rühren der behandelten Haare in einer Tensidlösung erhalten, die sich in einer verbleibenden Nasskämmkraftreduktion von 47% widerspiegelt. Haare behandelt mit einem nicht erfindungsgemäßen Rinse-Off Conditioner enthaltend die Emulsion **E1** (Vergleichsbeispiel **V-A2**) weisen eine leicht verringerte Nasskämmkraftreduktion von 85% auf. Nach der Tensidbehandlung erhält man jedoch eine im Vergleich zu Beispiel **A2** deutlich geringere Nasskämmkraftreduktion von 34%. Die Behandlung der Haare in Tensidlösung stellt eine Simulation mehrerer Haarwäschen mit Shampoo dar und demonstriert, dass der erfindungsgemäße Rinse-Off Conditioner eine bessere Waschbeständigkeit zeigt und die konditionierenden Eigenschaften länger erhalten bleiben als bei einer Haarbehandlung mit einem nicht erfindungsgemäßen Rinse-Off Conditioner.

**Vergleich der Silicondeponierung nach Behandlung von geschädigtem Haar mit Rinse-Off Conditioner und nach Simulierung mehrerer Shampoowäschen durch vierstündiges Rühren der Haare in einer wässrigen Tensidlösung**

    Beispiel **A2** - Vergleichsversuch **V-A2**
    Beispiel **A4** - Vergleichsversuch **V-A4**

In Analogie zur Bestimmung der Nasskämmkraftreduktion wurde die Effizienz der Siliconabscheidung auf geschädigtem Haar durch Behandlung mit Conditionern untersucht. Weiterhin wurde untersucht, wieviel des konditionierend wirkenden Organopolysiloxans auf der Haaroberfläche nach Simulation von Shampoowäschen durch vierstündiges Rühren der Haare in einer Tensidlösung verbleibt. Die Ergebnisse sind in Tabelle 5 zusammengefasst. Die Bestimmung der Silicondeponierung ist oben unter *Testmethoden* beschrieben.

**Tabelle 5:** Rinse-Off Conditioner / Silicondeponierung

| Bsp. / Vgl. | Silicondeponierung [ppm] | Silicondeponierung nach Rühren der Haare in einer wässrigen Tensidlösung (Persistenztest) [ppm] |
|---|---|---|
| A2 | 65 | 55 |
| V-A2 | 47 | 41 |
| A4 | 79 | 42 |
| V-A4 | 67 | 32 |

Tabelle 5 zeigt, dass die Deponierung des Silicons - auch nach Shampoowäschen - bei den erfindungsgemäßen Beispielen **A2** bzw. **A4** beständiger ist verglichen mit den nicht-erfindungsgemäßen Vergleichsversuchen **V-A2** bzw. **V-A4**.

**Farberhalt nach Behandlung von rot gefärbten Haaren mit Rinse-Off Conditioner und Simulierung mehrerer Shampoowäschen durch 10-minütiges Rühren der Haare in einer wässrigen Tensidlösung**

Beispiel **A2** - Vergleichsversuch **V-A2**

Der Farbschutz wurde an Haaren bestimmt, die unter Einsatz der Haarfarbe KOLESTON PERFECT® 77/44 der Firma WELLA rot gefärbt wurden (siehe oben unter *Testmethoden*). Die gefärbten Haare wurden anschließend mit einem erfindungsgemäßen Rinse-Off Conditioner analog Beispiel **A2** bzw. einem nicht erfindungsgemäßen Conditioner analog Vergleichsversuch **V-A2** behandelt. Die Ausgangsfarbe der Haare war nach Behandlung mit beiden Conditionern gleich. Zur Beurteilung der Farbschutzwirkung der aufgebrachten Conditioner wurden die Haare jeweils 10 Minuten in einer Tensidlösung geschüttelt, was einer Simulation von mehreren Shampoowäschen entspricht. Anschließend wurde die Farbänderung im Vergleich zur Ausgangsfarbe bestimmt (siehe Tabelle 6). Die Farbmessung und die Beurteilung des Farbschutzes sind oben unter *Testmethoden* beschrieben.

**Tabelle 6:** Rinse-Off Conditioner / Ergebnisse der Farbschutzmessungen nach Behandlung der gefärbten Haare mit einem erfindungsgemäßen Rinse-Off Conditioner im Vergleich zur Behandlung mit einem nicht-erfindungsgemäßen Conditioner, der keine Dispersion eines nicht vorvernetzten Organopolysiloxans enthält, und anschließender Simulierung mehrerer Shampoowäschen durch 10-minütiges Rühren der Haare in einer wässrigen Tensidlösung. Alle Ergebnisse im Vergleich zu frisch gefärbten Haar.

| Bsp./Vgl. | $\Delta$E | $\Delta$L |
|---|---|---|
| A2 | 5,7 | 3,6 |
| V-A2 | 8,0 | 6,2 |

Die Farbänderung durch Schütteln der Haare in Tensidlösung zur Simulation mehrerer Shampoowäschen ist bei Behandlung des Haares mit erfindungsgemäßem Conditioner **A2** mit $\Delta$E=5,7 geringer als beim nicht-erfindungsgemäßen Conditioner **V-A2** mit $\Delta$E=8,0. Die Änderung in der Helligkeit ist bei Haarbehandlung mit erfindungsgemäßem Conditioner **A2** mit $\Delta$L=3,6 geringer als bei der Haarbehandlung mit nicht-erfindungsgemäßem Conditioner **V-A2** mit $\Delta$L=6,2. Beide Parameter unterstreichen, dass die erfindungsgemäßen vorvernetzten Organopolysiloxane einen besseren Farbschutz ergeben als die unvernetzten Organopolysiloxane.

**Pflegeeigenschaften und Farbschutzwirkung von Conditionern, die mit erfindungsgemäßen Estern vorvernetzte Organopolysiloxane enthalten, im Vergleich zu solchen Conditionern, die mit Diethyloxalat (nicht-erfindungsgemäß) vorvernetzte Organopolysiloxane enthalten.**

**Vergleich der Rinse-Off Conditioner der erfindungsgemäßen Beispiele A1 und A3 mit dem Vergleichsversuch V-A5 (Diethyloxalat-Vernetzung)**

Die angeführten Beispiele und Vergleichsversuche unterscheiden sich dadurch, dass im Fall der Beispiele **A1** und **A3** wässrige Dispersionen von mit erfindungsgemäßen Estern vorvernetzten Organopolysiloxanen Einsatz fanden, im Vergleichsversuch **V-A5** analoge, aber mit Diethyloxalat (nicht-erfindungsgemäß) vorvernetzte Organopolysiloxane eingesetzt wurden. Der Aktivgehalt an Organopolysiloxan in der kosmetischen Zusammensetzung beträgt 2 Gew-%.

**Nasskämmkraft nach Behandlung von geschädigtem Haar mit Rinse-Off Conditioner und nach Simulierung mehrerer Shampoowäschen durch vierstündiges Rühren der Haare in einer wässrigen Tensidlösung**

Der Einsatz der erfindungsgemäßen wässrigen Dispersion **B1** im Rinse-Off Conditioner führt zu einer Verbesserung von konditionierenden Eigenschaften wie z.B. der Verringerung der Kämmkräfte im nassen Zustand im Vergleich zur entsprechenden nicht erfindungsgemäß mit Diethyloxalat vorvernetzten Organopolysiloxandispersion **V-A5.** Ziel der erfindungsgemäßen Rinse-Off Conditioner ist zusätzlich, dass die pflegenden Eigenschaften auch nach mehreren Shampoowäschen der Haare erhalten bleiben. Der Prozess der Shampoowäsche wird in diesem Beispiel durch vierstündiges Rühren von Rinse-Off-Conditioner behandelten Haarbündeln in einer Tensidlösung simuliert. Die Details dieser Behandlung sind oben unter *Testmethoden* beschrieben.

Die Ergebnisse sind nachfolgend für die Rinse-Off Conditioner des Beispiels **A1** sowie des Vergleichsversuchs **V-A5** in Tabelle 7 aufgeführt.

**Tabelle 7:** Rinse-Off Conditioner / Ergebnisse der Nasskämmkraftreduktion nach Behandlung mit einem erfindungsgemäßen Rinse-Off Conditioner im Vergleich zu einem nicht-erfindungsgemäßen Conditioner und nach Simulierung mehrerer Shampoowäschen durch vierstündiges Rühren der Haare in einer wässrigen Tensidlösung. Alle Ergebnisse beziehen sich auf den Vergleich zu unbehandelten Haartressen.

| Bsp./Vgl. | Reduktion Nasskämmkraft [%] | Reduktion Nasskämmkraft nach Rühren der Haare in einer wässrigen Tensidlösung [%] |
|---|---|---|
| A1 | 85 | 48 |
| V-A5 | 82 | 26 |

Durch Behandlung mit einem Rinse-Off Conditioner enthaltend eine wässrige Emulsion eines vorvernetzten Organopolysiloxans **B1** kann eine signifikante Reduktion der Nasskämmkraft der Haartressen von 85% gemessen werden. Insbesondere bleibt eine sehr hohe konditionierende Wirkung nach Rühren der behandelten Haare in einer Tensidlösung erhalten, die sich in einer Nasskämmkraftreduktion von 48% für Beispiel **A1** widerspiegelt. Haare behandelt mit einem nicht-erfindungsgemäßen Rinse-Off Conditioner analog Vergleichsversuch **V-A5** zeigen eine Reduktion der Nasskämmkraft von 82%, aber nach der Tensidbehandlung sinkt die Nasskämmkraftreduktion auf 26%. Die Behandlung der Haare in Tensidlösung stellt eine Simulation mehrerer Haarwäschen mit Shampoo dar und demonstriert, dass ein erfindungsgemäßer Rinse-Off Conditioner eine bessere Waschbeständigkeit zeigt und die konditionierenden Eigenschaften länger erhalten bleiben als bei einer Haarbehandlung mit einem nicht erfindungsgemäßen Rinse-Off Conditionern.

**Beispiel A5: Einsatz erfindungsgemäßer Emulsionen zur Vorbehandlung beim Haarfärben**

Das Färben von Haaren mit oxidativen Farben führt zu einer starken Schädigung der Haarfasern. Sowohl die Struktur des Haarinneren, als auch die Haaroberfläche wird durch den Haarfärbevorgang im Vergleich zu Naturhaar modifiziert, wodurch sich die kosmetischen Eigenschaften wie weicher Haargriff und Kämmbarkeit verschlechtern. Diesen negativen Effekten kann durch eine Vorbehandlung des Haares mit erfindungsgemäßen kosmetischen Formulierungen vor dem Färbeprozess entgegengewirkt werden.

Die Vorbehandlung erfolgte durch einminütiges Eintauchen von stark gebleichten Haaren (Klebetresse dicht aus Euro-Haar, Bleichstufe A) in eine 0,1 prozentige Lösung der Emulsion **B1** (aus Beispiel 1) in Wasser. Anschließend werden die Haare aus der wässrigen Lösung entnommen und fünf Minuten mit einem Haartrockner geföhnt. Dieser Vorbehandlung schließt sich der oben unter *Testmethoden* beschriebene Färbeprozess unter Einsatz der Haarfarbe L'Oreal Majirel MIX ROUGE an.

Nach dem Färben und Trocknen der Haare bei 22°C und 50% Luftfeuchtigkeit wurde in einem Paneltest die Weichheit von vorbehandelten Haaren verglichen mit Haaren, die vor dem Färbeprozess nicht behandelt wurden. Die vorbehandelten Haare wurden durchgehend als weicher beurteilt. Ein Farbunterschied zwischen vorbehandelten und nicht-vorbehandelten Haaren nach dem Färbeprozess konnte optisch nicht festgestellt werden. Im Anschluss wurden sowohl die vorbehandelten, als auch die nicht-vorbehandelten Haare zur Simulierung mehrerer Shampoowäschen in einer wässrigen Tensidlösung wie oben (unter *Testmethoden*) beschrieben für zehn Minuten gerührt. Nach dem Trocknen wurde der Paneltest zur Beurteilung der Weichheit der Haartressen wiederholt. Auch nach der Tensidbehandlung wurden die vorbehandelten Haare durchgehend als weicher beurteilt. Dies indiziert eine hohe Persistenz des kosmetischen Effekts nach einer Vorbehandlung der Haare mit erfindungsgemäßen kosmetischen Emulsionen bzw. Zusammensetzungen.

Zusätzlich wurde die Farbänderung ΔE der gefärbten Haare nach der Tensidbehandlung im Vergleich zum Ausgangswert mit Hilfe des Farbmessgeräts Spectro Guide der Firma Byk-Gardner bestimmt. Die Farbänderung ΔE = 4,6 ist bei den vorbehandelten Haartressen geringer als bei nicht vorbehandelten Haartressen mit ΔE = 7,3. Eine Vorbehandlung der Haare mit erfindungsgemäßen kosmetischen Emulsionen bzw. Zusammensetzungen führt zu einem besseren Farberhalt.

**Beispiele A6 und A7 sowie Vergleichsversuch V-A6** Kosmetische Zusammensetzung: Shampoo

Die nachfolgenden Beispiele repräsentieren erfindungsgemäße kosmetische Zusammensetzungen enthaltend die Emulsionen **B4** aus Beispiel 4 und **B7** aus Beispiel 7. Das Vergleichsbeispiel **V-A6** repräsentiert eine marktübliche Shampooformulierung enthaltend eine Emulsion eines Dimethicons (Dimethylpolysiloxans; BELSIL® DM 5102 E, erhältlich bei der Wacker Chemie AG) mit einer Viskosität von 60.000 mm²/s (bei 25°C).

Der Aktivgehalt an Organopolysiloxan in der kosmetischen Zusammensetzung beträgt 1,3 %.

Die Zusammensetzung der Shampoos ist in der Tabelle 8 zusammengefasst.

**Herstellanweisung:**

32,11 Teile Wasser werden vorgelegt und unter Rühren auf 50°C erwärmt. Dabei werden 0,20 Teile Guar Hydroxylpropyltrimonium Chloride, 6,06 Teile Sodium Lauryl Sulfate, 29,90 Teile Sodium Laureth Sulfate, 0,05 Teile Citric Acid und 5,0 Teile Cocamidopropyl Betaine zugegeben. Die Mischung wird gerührt bis 50°C erreicht sind und die Inhaltsstoffe gelöst vorliegen. Dann wird die Mischung abgekühlt. In einem separaten Gefäß werden 20,0 Teile Wasser vorgelegt, unter Rühren 0,60 Teile Carbomer zugegeben und gerührt bis eine homogene Mischung entsteht. Nun werden 0,06 Teile Lactic Acid zugegeben. Diese Mischung wird zur ersten Mischung zugegeben. Bei 40°C werden 0,95 Teile Phenoxyethanol, Ethylhexylglycerin zugegeben. Unter weiterem Rühren erfolgt die Zugabe von 0,30 Teilen C12-13 Alkyl Lactate, 3,71 Teilen der erfindungsgemäßen Emulsionen **B4** und **B7** bzw. 2,60 Teilen der nicht-erfindungsgemäßen Dimethicon-Emulsion BELSIL® DM 5102 E, 0,40 Teilen Sodium Hydroxide und 0,66 Teilen Sodium Chloride. Der erforderliche pH-Wert von 6,5 ist bei Bedarf durch Zugabe von Sodium Hydroxide einzustellen.

**Tabelle 8:** Shampooformulierungen **A6**, **A7** und **V-A6**

| Bestandteile (INCI-Name) | Beispiel **A6** [Gew.-teile] | Beispiel **A7** [Gew.-teile] | Vergleichsversuch **V-A6** [Gew.-teile] |
|---|---|---|---|
| Citric Acid[1) | 0,05 | 0, 05 | 0,05 |
| Cocamidopropyl Betaine[2) | 5, 00 | 5, 00 | 5,00 |
| Sodium Laureth Sulfate[3) | 29,90 | 29,90 | 29,90 |
| Guar Hydroxypropyltrimonium Chloride[4) | 0,20 | 0,20 | 0,20 |
| Sodium Lauryl Sulfate[5) | 6,06 | 6,06 | 6,06 |
| Aqua (DI Water) | 32,11 | 32,11 | 32,11 |
| Carbomer[6) | 0,60 | 0,60 | 0,60 |
| Lactic Acid[7) | 0,06 | 0,06 | 0,06 |
| Aqua (DI Water) | 20,00 | 20,00 | 20,00 |
| Phenoxyethanol, Ethylhexylglycerin[8) | 0,95 | 0,95 | 0,95 |
| C12-13 Alkyl Lactate[9) | 0,30 | 0,30 | 0,30 |
| Emulsion **B4** aus Bsp.4 | 3,71 | | |
| Emulsion **B7** aus Bsp.7 | | 3,71 | |
| BELSIL® DM 5102 E[10) (Dimethicon-Emulsion) | | | 2,60 |
| Sodium Hydroxide[11) | 0,40 | 0,40 | 0,40 |
| Sodium Chloride[12) | 0,66 | 0,66 | 0,66 |

1) Citric Acid: Citric Acid, Sigma 2) Cocamidopropyl Betaine: Genagen CAB 818 30%, Clariant 3) Sodium Laureth Sulfat: Genapol® LRO 26,5%, Clariant 3) Glycol Distearate: Genapol® PMS, Clariant GmbH 4) Guar Hydroxypropyltrimonium Chloride: N-Hance® BF 13, Ashland 5) Sodium Lauryl Sulfat: Texapon K 12 G, BASF 6) Carbomer: Carbopol 980, Lubrizol 7) Lactic acid: Z-(+)Milchsäure, 90%, Bernd Kraft GmbH 8) Phenoxyethanol, Ethylhexylglycerin: Euxyl PE 9010, Schülke & Mayr 9) C12-13 Alkyl Lactate: Ceraphyl™ 41 ester, Ashland 10) BELSIL® DM 5102 E, erhältlich bei der Wacker Chemie AG 11)Sodium Hydroxide: Natriumhydroxid, Sigma-Aldrich 12)Sodium Chloride: Natriumchlorid reinst, Bernd Kraft GmbH

**Vergleich der Shampoos der erfindungsgemäßen Beispiele A6 und A7 mit dem Vergleichsversuch V-A6**

Die angeführten Beispiele und Vergleichsversuche unterscheiden sich dadurch, dass im Fall der Beispiele **A6** und **A7** wässrige Dispersionen von vorvernetzten Organopolysiloxanen Einsatz fanden. Im Vergleichsversuch **V-A6** wird die handelsübliche, wässrige Dimethylpolysiloxan-Emulsion BELSIL® DM 5102 E (Wacker Chemie AG) eingesetzt, die als konditionierenden Wirkstoff ein Dimethicon (Dimethylpolysiloxan) mit der Viskosität von 60.000 $mm^2/s$ enthält. Der Aktivgehalt an Organopolysiloxan in den kosmetischen Zusammensetzungen beträgt 1,3 Gew-%.

Die Ergebnisse der kosmetischen Wirkung der Shampoos sind in der Tabelle 9 zusammengefasst.

**Tabelle 9:** Shampoo / Ergebnisse der Nasskämmkraftreduktion. Verbesserung der Weichheit und abgeschiedenen Siliconmenge auf geschädigtem Haar nach Behandlung mit Shampoo. Alle Ergebnisse im Vergleich zu unbehandelten Haartressen.

| Bsp./Vgl. | Reduktion Nasskämmkraft [%] | Verbesserung Weichheit [%] | Siliconabscheidung auf der Haaroberfläche [ppm] |
|---|---|---|---|
| Bsp. A6 | 67 | 28 | 126 |
| Bsp. A7 | 68 | 55 | 140 |
| Vergleichsversuch V-A6*) | 20 | 2 | 46 |
| *) BELSIL® DM 5102 E (Dimethicon-Emulsion), erhältlich bei der Wacker Chemie AG | | | |

Die Shampoos gemäß den Beispielen **A6** und **A7** mit den erfindungsgemäßen Emulsionen zeigen eine wesentlich höhere Reduktion der Nasskämmkraft, eine deutlich verbesserte Weichheit (gemäß Zug-Prüfung) und eine wesentlich höhere Deponierung des Silicons auf den Haaren im Vergleich zu dem Shampoo gemäß Vergleichsversuch **V-A6** mit der handelsüblichen Dimethicon-Emulsion.

**Beispiele A8 und A9: Kosmetische Zusammensetzung - Shampoo**

Das nachfolgende Beispiel **A8** repräsentiert eine kosmetische Zusammensetzung enthaltend die Emulsionen **B4** aus Beispiel 4, das Beispiel **A9** ist eine kosmetische Zusammensetzung enthaltend die Emulsion **B1** in Kombination mit der Dimethylpolysiloxan-Emulsion BELSIL® DM 5102 E (erhältlich bei der Wacker Chemie AG). Der Aktivgehalt an Organopolysiloxan in den kosmetischen Zusammensetzungen beträgt 1,3 %.

Die Zusammensetzungen der Shampoos sind in der Tabelle 10 zusammengefasst.

Herstellanweisung:

0,30 Teile Guar Hydroxypropyltrimonium Chloride werden in Wasser dispergiert. 41,50 Teile Sodium Laureth Sulfate werden langsam eingerührt und die Mischung wird schrittweise auf 75°C erwärmt. Während des Erwärmens werden bei 50°C 0,20 Teile PEG-150 Distearate zugegeben, bei 65°C erfolgt die Zugabe von 0,50 Teilen Glycol Distearate. Anschließend wird die Mischung abgekühlt. Bei Erreichen von 35°C werden 0,90 Teile Phenoxyethanol, Ethylhexylglycerin und die Emulsionen entsprechend der Beispiele zugegeben und 5 Minuten gerührt. Abschließend werden 13,4 Teile Cocamidopropyl Betaine zugegeben und weitere 10 Minuten gerührt.

Die Verbesserung der Weichheit von geschädigten Haartressen (im Vergleich zu unbehandelten Haartressen) nach Behandlung mit den Shampooformulierungen gemäß den Beispielen **A8** und **A9** wurde bestimmt. Die Ergebnisse sind in Tabelle 11 zusammengefasst.

**Tabelle 10:** Shampooformulierungen **A8** und **A9**

| (Angabe in Gew.-teilen) | | |
|---|---|---|
| **Bestandteile (INCI-name)** | **Bsp. A8** | **Bsp. A9** |
| Aqua (Water VES) | ad 100 | ad 100 |
| Guar Hydroxy-propyltrimonium Chloride [1] | 0,30 | 0, 30 |
| Sodium Laureth Sulfate [2] | 41,50 | 41,50 |
| Glycol Distearate [3] | 0,50 | 0,50 |

(fortgesetzt)

| (Angabe in Gew.-teilen) | | |
|---|---|---|
| **Bestandteile (INCI-name)** | **Bsp. A8** | **Bsp. A9** |
| PEG-150 Distearate [4] | 0,20 | 0,20 |
| Emulsion **B1** aus Beispiel 1 | 1, 43 | |
| BELSIL® DM 5102 E [5] (Dimethicon-Emulsion) | 1, 60 | |
| Emulsion **B4** aus Beispiel 4 | | 3, 71 |
| Cocamidopropyl Betaine [6] | 13,33 | 13,33 |
| Phenoxyethanol, Ethylhexylglycerin [7] | 0, 90 | 0, 90 |
| 1) Guar Hydroxypropyltrimonium Chloride: N-Hance® 3196, Ashland. <br> 2) Sodium Laureth Sulfat: Genapol® LRO 26,5%, Clariant GmbH <br> 3) Glycol Distearate: HALLSTAR® EGDS, The Hallstar Company <br> 4) PEG-150 Distearate: Eumulgin® EO 33, BASF AG <br> 5) BELSIL® DM 5102 E, erhältlich bei der Wacker Chemie AG <br> 6) Cocamidopropyl Betaine: Genagen® CAB 30%, Clariant GmbH <br> 7) Phenoxyethanol, Ethylhexylglycerin: Euxyl PE 9010, Schülke & Mayr | | |

**Tabelle 11:** Shampoo / Verbesserung der Weichheit. Alle Ergebnisse im Vergleich zu unbehandelten Haartressen.

| Bsp. | Verbesserung Weichheit [%] |
|---|---|
| **A8** | 45 |
| **A9** | 46 |

Geschädigte Haare, die mit den Shampoos aus den Beispielen **A8** und **A9** behandelt wurden, werden in einem Test zur Beurteilung der Weichheit (gemäß Zug-Prüfung) als deutlich weicher beurteilt als geschädigte, unbehandelte Haare.

**Beispiel A10:** Kosmetische Zusammensetzung - Shampoo

Das nachfolgende Beispiel repräsentiert eine kosmetische Zusammensetzungen enthaltend die Emulsion **B7** aus Beispiel 7. Der Aktivgehalt an Organopolysiloxan in der kosmetischen Zusammensetzung beträgt 1,0 %.
Die Zusammensetzung des Shampoos ist in der Tabelle 12 zusammengefasst.

**Tabelle 12:** Shampooformulierung **A10**

| (Angabe in Gew.-teilen) | |
|---|---|
| **Bestandteile (INCI-name)** | **Bsp. A10** |
| Aqua (Water VES) | ad 100 |
| Polyquaternium-10 [1] | 0,10 |
| Sodium Laureth Sulfate [2] | 52,80 |
| PEG-150 Distearate [3] | 0,25 |
| Cocamide MEA [4] | 1,00 |
| Emulsion **B7** aus Beispiel 7 | 2,85 |
| Cocamidopropyl Betaine [5] | 10,06 |

(fortgesetzt)

| (Angabe in Gew.-teilen) | |
|---|---|
| **Bestandteile (INCI-name)** | **Bsp. A10** |
| Phenoxyethanol, Ethylhexylglycerin [6] | 0, 95 |
| 1) Polyquaternium-10: UCARE Polymer JR 400, Dow Chemical.<br>2) Sodium Laureth Sulfat: Genapol® LRO 26,5%, Clariant GmbH<br>3) PEG-150 Distearate: Eumulgin® EO 33, BASF AG<br>4) Cocamide MEA: Comperlan 100, BASF AG<br>5) Cocamidopropyl Betaine: Genagen® CAB 30%, Clariant GmbH<br>7) Phenoxyethanol, Ethylhexylglycerin: Euxyl PE 9010, Schülke & Mayr | |

Geschädigte Haare, die mit dem Shampoo aus Beispiel **A10** behandelt wurden, sind weicher (gemäß Panel-Test) als geschädigte, unbehandelte Haare.

**Beispiel A11** - **A14**

Die nachfolgenden Beispiele repräsentieren erfindungsgemäße kosmetische Zusammensetzungen **A11** - **A14** gemäß Tabelle 13 enthaltend die Emulsionen **B4, B5, B6 und B7** aus den Beispielen 4 bis 7. Der Aktivgehalt an vorvernetztem Organopolysiloxan in der kosmetischen Zusammensetzung beträgt 2 %.

**Tabelle 13:** Rinse-Off Conditioner **A11** bis **A14**

| Bestandteile (INCI-name) | Beispiel A11 [Gew. - teile] | Beispiel A12 [Gew.-teile] | Beispiel A13 [Gew.-teile] | Beispiel A14 [Gew.-teile] |
|---|---|---|---|---|
| Water | ad 100 | ad 100 | ad 100 | ad 100 |
| Hydroxyethylcellulose[1] | 1,3 | 1,3 | 1,3 | 1,3 |
| Cetyl Alcohol[2] | 0,5 | 0,5 | 0,5 | 0, 5 |
| Polysorbate 80[3] | 0,5 | 0,5 | 0,5 | 0, 5 |
| Behentrimonium Chloride[4] | 0,2 | 0,2 | 0,2 | 0,2 |
| Stearyl Alcohol[5] | 0,5 | 0,5 | 0,5 | 0, 5 |
| Citric Acid[6] | 0,1 | 0,1 | 0,1 | 0,1 |
| Tetrasodium EDTA[7] | 0,2 | 0,2 | 0,2 | 0,2 |
| Emulsion **B4** aus Bsp. 4 | 5,71 | | | |
| Emulsion **B5** aus Bsp. 5 | | 5, 71 | | |
| Emulsion **B6** aus Bsp. 6 | | | 5,71 | |
| Emulsion **B7** aus Bsp. 7 | | | | 5,71 |
| Phenoxyethanol, Ethylhexylglycerin[8] | 0,9 | 0, 9 | 0, 9 | 0, 9 |

Die in Tabelle 13 genannten Rohstoffe sind unter folgenden Handelsnamen erhältlich:

1)Hydroxyethylcellulose: Natrosol 250 HR, Ashland
2)Cetylalkohol: Cetylalkohol, Merck KGaA
3) Polysorbate 80: Tween™ 80, Croda GmbH
4) Behentrimonium Chloride: Genamin® KDMP, Clariant GmbH
5) Stearylalkohol: Stearylalkohol, Merck KGaA
6)Citric Acid: Citric Acid, Sigma
7)Tetrasodium EDTA: EDETA® B Pulver, BASF Corporation
8)Phenoxyethanol, Ethylhexylglycerin: Euxyl PE 9010, Schülke & Mayr

Herstellanweisung:

Wasser wird vorgelegt und unter Rühren auf 75°C erwärmt. Dabei werden 1,3 Teile Hydroxyethylcellulose zugegeben. Wenn 65°C erreicht sind, werden 0,5 Teile Polysorbate 80, 0,5 Teile Stearyl Alcohol, 0,5 Teil Cetyl Alcohol und 0,2 Teile Behentrimonium Chloride zugegeben. Die Mischung wird gerührt bis 75°C erreicht sind und die Inhaltsstoffe gelöst vorliegen. Dann wird die Mischung abgekühlt. Während des Abkühlens werden 0,1 Teile Citric Acid und 0,2 Teile Tetrasodium EDTA zugegeben. Bei 35°C werden 0,9 Teile Phenoxyethanol, Ethylhexylglycerin zugegeben. Unter weiterem Rühren erfolgt die Zugabe der Emulsion aus den Beispielen. Die Zusammensetzung wird 15 Minuten unter Rühren homogenisiert.

**Nasskämmkraft nach Behandlung von geschädigtem Haar mit Rinse-Off Conditioner und nach Simulierung mehrerer Shampoowäschen durch vierstündiges Rühren der Haare in einer wässrigen Tensidlösung**

Tabelle 14: Rinse-Off Conditioner / Ergebnisse der Nasskämmkraftreduktion nach Behandlung mit einem erfindungsgemäßen Rinse-Off Conditioner und nach Simulierung mehrerer Shampoowäschen durch vierstündiges Rühren der Haare in einer wässrigen Tensidlösung. Alle Ergebnisse beziehen sich auf den Vergleich zu unbehandelten Haartressen.

| Bsp. | Reduktion Nasskämmkraft nach Conditionerbehandlung [%] | Reduktion Nasskämmkraft nach Conditionerbehandlung und anschließendem Rühren der Haare in einer wässrigen Tensidlösung (Persistenztest) [%] |
|---|---|---|
| A11 | 78 | 37 |
| A12 | 82 | 63 |
| A13 | 83 | 69 |
| A14 | 87 | 44 |

Durch Behandlung mit Rinse-Off Conditionern enthaltend eine wässrige Emulsion eines vorvernetzten Organopolysiloxans **B4, B5, B6** bzw. **B7** kann eine signifikante Reduktion der Nasskämmkraft der Haartressen im Bereich 78% bis 87% erreicht werden. Insbesondere bleibt eine sehr hohe konditionierende Wirkung nach Rühren der behandelten Haare in einer Tensidlösung erhalten, die sich in einer Nasskämmkraftreduktion von 37% für Beispiel **A11,** 44% für Beispiel **A14** und Werten von 63% und 69% für die Beispiele A12 und A13 widerspiegelt.

**Beispiel A15** - **Rinse-Off Conditioner**

Das nachfolgende Beispiel repräsentiert eine erfindungsgemäße kosmetische Zusammensetzung **A15** gemäß Tabelle 15 enthaltend die Emulsionen **B3** aus Beispiel 3. Der Aktivgehalt an vorvernetztem Organopolysiloxan in der kosmetischen Zusammensetzung beträgt 0,5 %.

Herstellanweisung:

Wasser wird vorgelegt und unter Rühren auf 75°C erwärmt. Dabei werden 1,3 Teile Hydroxyethylcellulose zugegeben. Wenn 65°C erreicht sind, werden 0,5 Teile Stearamidopropyl Dimethylamine, 1,0 Teile Polysorbate 80, 3,0 Teile Stearyl Alcohol, 2,0 Teile Cetyl Alcohol und 1,8 Teile Behentrimonium Chloride zugegeben. Die Mischung wird gerührt bis 75°C erreicht sind und die Inhaltsstoffe gelöst vorliegen. Dann wird die Mischung abgekühlt. Während des Abkühlens werden 0,2 Teile Citric Acid und 0,2 Teile Tetrasodium EDTA zugegeben. Bei 35°C werden 0,9 Teile Phenoxyethanol, Ethylhexylglycerin zugegeben. Unter weiterem Rühren erfolgt die Zugabe der Emulsion aus dem Beispiel. Die Zusammensetzung wird 15 Minuten unter Rühren homogenisiert.

**Tabelle 15:** Rinse-Off Conditioner **A15**

| Bestandteile (INCI-name) | Beispiel **A15** [Gew.-teile] |
|---|---|
| Water | ad 100 |

(fortgesetzt)

| Bestandteile (INCI-name) | Beispiel **A15** [Gew.-teile] |
|---|---|
| Hydroxyethyl-cellulose [1] | 1,3 |
| Cetyl Alcohol [2] | 2,0 |
| Polysorbate 80 [3] | 1,0 |
| Behentrimonium Chloride [4] | 1,8 |
| Stearamidopropyl Dimethylamine [5] | 0,5 |
| Stearyl Alcohol [6] | 3,0 |
| Citric Acid [7] | 0,2 |
| Tetrasodium EDTA [8] | 0,2 |
| Emulsion **B3** aus Bsp. 3 | 1,87 |
| Phenoxyethanol, Ethylhexylglycerin [9] | 0, 9 |

Die in Tabelle 15 genannten Rohstoffe sind unter folgenden Handelsnamen erhältlich:

1) Hydroxyethylcellulose: Natrosol 250 HR, Ashland
2) Cetylalkohol: Cetylalkohol, Merck KGaA
3) Polysorbate 80: Tween™ 80, Croda GmbH
4) Behentrimonium Chloride: Genamin® KDMP, Clariant GmbH
5) Stearamidopropyl Dimethylamine, Incromine™ SB, Croda GmbH
6) Stearylalkohol: Stearylalkohol, Merck KGaA
7) Citric Acid: Citric Acid, Sigma
8) Tetrasodium EDTA: EDETA® B Pulver, BASF Corporation
9) Phenoxyethanol, Ethylhexylglycerin: Euxyl PE 9010, Schülke & Mayr

Durch Behandlung geschädigter Haare mit dem erfindungsgemäßen Rinse-Off Conditioner aus Beispiel **A15,** enthaltend eine wässrige Emulsion eines vorvernetzten Organopolysiloxans **B3** (Beispiel 3) kann eine deutliche Reduktion der Nasskämmkraft der Haartressen (im Vergleich zu unbehandelten Haartressen) erzielt werden.

**Patentansprüche**

1.   Wässrige Dispersionen, vorzugsweise wässrige Emulsionen, enthaltend vorvernetzte Organopolysiloxane,

   die durchschnittlich mindestens eine Struktureinheit der allgemeinen Formel

   $$SiRO_{2/2}\text{-}Y\text{-}SiRO_{2/2} \qquad (I)$$

   und Einheiten der Formel

   $$R_2SiO_{2/2} \qquad (II),$$

   enthalten, wobei

   Y ein Rest der Formel

   $$-R^2\text{-}[NR^3\text{-}R^4\text{-}]_x\text{-}NR^3\_OC\text{-}[C(Z^1)(H)]_{k1}\text{-}[C(Z^2)(H)]_{k2}\text{-}CO\text{-}NR^3\text{-}[R^4\text{-}NR^3]_x\text{-}R^2\text{-}$$

   bedeutet,
   R gleich oder verschieden sein kann, ein einwertiger Kohlenwasserstoffrest mit 1 bis 18 Kohlenstoffatomen bedeutet, der ein oder mehrere Heteroatome aus der Gruppe N, P, S, O, und Halogen enthalten kann,
   $R^2$ ein SiC-gebundener, zweiwertiger linearer oder verzweigter Kohlenwasserstoffrest mit 3 bis 18 Kohlenstoffatomen, vorzugsweise ein Alkylenrest mit 3 bis 10 Kohlenstoffatomen, bedeutet,
$R^3$ ein Wasserstoffatom, einen Alkylrest mit 1 bis 8 Kohlenstoffatomen oder einen Acylrest, wie Acetylrest, bedeutet, vorzugsweise ein Wasserstoffatom ist,
$R^4$ ein zweiwertiger Kohlenwasserstoffrest mit 1 bis 6 Kohlenstoffatomen, vorzugsweise ein Alkylenrest mit 1 bis 6 Kohlenstoffatomen, bedeutet,
k1 0, 1, 2 oder 3 ist,
k2 0, 1, 2 oder 3 ist,
x 0, 1, 2, 3 oder 4, vorzugsweise 0 oder 1 ist,
$Z^1$ -OH, H oder -NHR$^3$ bedeutet,
$Z^2$ -OH, H oder -NHR$^3$ bedeutet,

mit der Maßgabe, dass die Summe k1+k2≥1 ist und dass mindestens ein Rest $Z^1$ oder $Z^2$ eine Hydroxy- oder NHR$^3$-Gruppe, vorzugsweise eine Hydroxygruppe, ist, in der verbrückenden Gruppe Y damit mindestens eine Hydroxy- oder NHR$^3$-Gruppe, vorzugsweise mindestens eine Hydroxygruppe, enthalten ist.

2. Wässrige Dispersionen nach Anspruch 1, **dadurch gekennzeichnet, dass** die vorvernetzten Organopolysiloxane Siloxaneinheiten der Formel

$$R_{3-d}(OR^1)_d SiO_{1/2} \qquad (III),$$

enthalten, wobei

R die in Anspruch 1 dafür angegebene Bedeutung hat,
$R^1$ gleich oder verschieden sein kann, ein Wasserstoffatom oder einen Alkylrest mit 1 bis 18 Kohlenstoffatomen bedeutet, der durch ein oder mehrere separate Sauerstoffatome unterbrochen sein kann, und
d 0 oder 1 ist.

3. Wässrige Dispersionen nach Anspruch 1 oder 2, **dadurch gekennzeichnet, dass** Y ein Rest der Formel

$$-R^2-[NH-CH_2CH_2-]_{x'}-NH\_OC-CH(OH)-CH(OH)-CO-NH-[CH_2CH_2-NH]_{x'}-R^2-$$

ist, wobei

x' 0 oder 1 ist und
$R^2$ ein Rest der Formel -(CH$_2$)$_3$- oder -CH$_2$-CH(CH$_3$)-CH$_2$- ist.

4. Verfahren zur Herstellung der wässrigen Dispersionen, vorzugsweise wässrigen Emulsionen, von vorvernetzten Organopolysiloxanen, indem

wässrige Dispersionen, vorzugsweise wässrige Emulsionen von Aminoorganopolysiloxanen (1) der Formel

$$(R^1O)_d A_e R_{3-d-e} SiO(SiARO)_p (SiR_2O)_q SiR_{3-d-e}A_e (OR^1)_d \qquad (IV),$$

wobei

A ein Aminorest der allgemeinen Formel

$$-R^2-[NR^3-R^4-]_x NR^3{}_2 ,$$

ist,

R gleich oder verschieden sein kann, ein einwertiger Kohlenwasserstoffrest mit 1 bis 18 Kohlenstoffatomen bedeutet, der ein oder mehrere Heteroatome aus der Gruppe N, P, S, O, und Halogen enthalten kann,
$R^1$ gleich oder verschieden sein kann, ein Wasserstoffatom oder einen Alkylrest mit 1 bis 18 Kohlenstoffatomen bedeutet, der durch ein oder mehrere separate Sauerstoffatome unterbrochen sein kann,
$R^2$ ein SiC-gebundener, zweiwertiger linearer oder verzweigter Kohlenwasserstoffrest mit 3 bis 18 Kohlenstoffatomen, vorzugsweise ein Alkylenrest mit 3 bis 10 Kohlenstoffatomen, bedeutet,

$R^3$ ein Wasserstoffatom, einen Alkylrest mit 1 bis 8 Kohlenstoffatomen oder einen Acylrest, wie Acetylrest, bedeutet, vorzugsweise ein Wasserstoffatom ist,

$R^4$ ein zweiwertiger Kohlenwasserstoffrest mit 1 bis 6 Kohlenstoffatomen, vorzugsweise ein Alkylenrest mit 1 bis 6 Kohlenstoffatomen, bedeutet,

d 0 oder 1 ist,

e 0 oder 1 ist,

p eine ganze Zahl von mindestens 1 und höchstens 1000 ist und

q 0 oder eine ganze Zahl von 1 bis 2000 ist,

x 0, 1, 2, 3 oder 4, vorzugsweise 0 oder 1 ist,

mit reaktiven Estern (2) der Formel

$$R^5O_2C\text{-}[C(Z^1)(H)]_{k1}\text{-}[C(Z^2)(H)]_{k2}\text{-}CO_2R^5... \qquad (V),$$

wobei

$R^5$ gleich oder verschieden sein kann und einen O-gebundenen, gesättigten oder ungesättigten, linearen oder verzweigten, einwertigen Kohlenwasserstoffrest mit 1-20 Kohlenstoffatomen je Rest, der ein oder mehrere Heteroatome aus der Gruppe N, P, S, O und Halogen enthalten kann, bedeutet, k1 0, 1, 2 oder 3 ist, k2 0, 1, 2 oder 3 ist,

$Z^1$ -OH, H oder -$NHR^3$ bedeutet,

$Z^2$ -OH, H oder -$NHR^3$ bedeutet,

mit der Maßgabe, dass die Summe $k1+k2 \geq 1$ ist und dass mindestens ein Rest $Z^1$ oder $Z^2$ eine Hydroxy- oder $NHR^3$-Gruppe, vorzugsweise eine Hydroxygruppe, ist, umgesetzt werden.

5. Verfahren nach Anspruch 4, **dadurch gekennzeichnet, dass** A ein Aminorest der Formel

$$-R^2\text{-}[NH\text{-}CH_2CH_2\text{-}]_{x'}\text{-}NH_2$$

ist, wobei

x' 0 oder 1 ist und

$R^2$ ein Rest der Formel $-(CH_2)_3-$ oder $-CH_2-CH(CH_3)-CH_2-$ ist.

6. Verfahren nach Anspruch 4 oder 5, **dadurch gekennzeichnet, dass** die reaktiven Ester (2) Dimethyltartrate und Diethyltartrate sind.

7. Vorvernetzte Organopolysiloxane, die durchschnittlich mindestens eine Struktureinheit, vorzugsweise mindestens zwei Struktureinheiten, bevorzugt mindestens drei Struktureinheiten, der allgemeinen Formel

$$SiRO_{2/2}\text{-}Y\text{-}SiRO_{2/2} \qquad (I)$$

und Einheiten der Formel

$$R_2SiO_{2/2} \qquad (II),$$

enthalten, wobei

Y ein Rest der Formel

$$-R^2\text{-}[NR^3\text{-}R^4\text{-}]_x\text{-}NR^3\_OC\text{-}[C(Z^1)(H)]_{k1}\text{-}[C(Z^2)(H)]_{k2}\text{-}CO\text{-}NR^3\text{-}[R^4\text{-}NR^3]_x\text{-}R^2\text{-}$$

bedeutet,

R, $R^2$, $R^3$, $R^4$, k1, k2, $Z^1$ und $Z^2$ die in Anspruch 1 dafür angegebene Bedeutung haben, mit der Maßgabe, dass die Summe $k1+k2 \geq 1$ ist und dass mindestens ein Rest $Z^1$ oder $Z^2$ eine Hydroxy- oder $NHR^3$-Gruppe, vorzugsweise eine Hydroxygruppe, ist,

in der verbrückenden Gruppe Y damit mindestens eine Hydroxy- oder NHR$^3$-Gruppe, vorzugsweise mindestens eine Hydroxygruppe, enthalten ist.

8. Vorvernetzte Organopolysiloxane nach Anspruch 7, **dadurch gekennzeichnet, dass** sie Siloxaneinheiten der Formel

$$R_{3-d}(OR^1)_d SiO_{1/2} \qquad \text{(III)}$$

enthalten, wobei

R und Y die in Anspruch 1 dafür angegebene Bedeutung haben und
d und R$^1$ die in Anspruch 2 dafür angegebene Bedeutung haben.

9. Verfahren zur Herstellung der vorvernetzten Organopolysiloxane, **dadurch gekennzeichnet, dass** Aminoorgano-polysiloxanen der Formel

$$(R^1O)_d A_e R_{3-d-e} SiO(SiARO)_p (SiR_2O)_q SiR_{3-d-e} A_e (OR^1)_d \qquad \text{(IV)}$$

mit reaktiven Estern der Formel

$$R^5O_2C\text{-}[C(Z^1)(H)]_{k1}\text{-}[C(Z^2)(H)]_{k2}\text{-}CO_2R^5... \qquad \text{(V)}$$

wobei

A, R, R$^1$, R$^5$, k1, k2, Z$^1$ und Z$^2$ die in Anspruch 4 dafür angegebene Bedeutungen haben,
mit der Maßgabe, dass die Summe k1+k2≥1 ist und dass mindestens ein Rest Z$^1$ oder Z$^2$ eine Hydroxy-oder NHR$^3$-Gruppe, vorzugsweise eine Hydroxygruppe, ist,
umgesetzt werden,
und die so erhaltenen vorvernetzten Organopolysiloxane gegebenenfalls anschließend in Wasser emulgiert werden.

10. Kosmetische Zusammensetzungen enthaltend wässrige Dispersionen, vorzugsweise wässrige Emulsionen, von vorvernetzten Organopolysiloxanen nach Anspruch 1, 2 oder 3 oder hergestellt nach Anspruch 4, 5 oder 6 oder vorvernetzte Organopolysiloxane nach Anspruch 7 oder 8 oder hergestellt nach Anspruch 9.

11. Kosmetische Zusammensetzungen nach Anspruch 10, **dadurch gekennzeichnet, dass** sie Konditionierungsmittel enthalten.

12. Verwendung der kosmetischen Zusammensetzungen nach Anspruch 10 oder 11 zur Behandlung von keratinischen Fasern, vorzugsweise zur Reinigung und Pflege von keratinischen Fasern.

13. Verwendung der kosmetischen Zusammensetzungen nach Anspruch 10 oder 11 zum Konditionieren von keratinischen Fasern, insbesondere um die Kämmbarkeit von keratinischen Fasern zu erleichtern.

14. Verwendung nach Anspruch 12 oder 13, **dadurch gekennzeichnet, dass** die keratinischen Fasern Haare sind.

15. Verfahren zur Behandlung von keratinhaltigen Fasern, vorzugsweise Haaren, indem die kosmetischen Zusammensetzungen nach Anspruch 10 oder 11 auf die keratinischen Fasern, vorzugsweise Haare, aufgetragen werden und dann gegebenenfalls mit Wasser gespült werden.

**Claims**

1. Aqueous dispersions, preferably aqueous emulsions, comprising precrosslinked organopolysiloxanes which contain on average at least one structural unit of the general formula

$$SiRO_{2/2}\text{-Y-}SiRO_{2/2} \qquad \text{(I)}$$

and units of the formula

$$R_2SiO_{2/2} \qquad (II),$$

where

Y is a radical of the formula

$$-R^2-[NR^3-R^4-]_x-NR^3\_OC-[C(Z^1)(H)]_{k1}-[C(Z^2)(H)]_{k2}-CO-NR^3-[R^4-NR^3]_x-R^2,$$

R may be identical or different and denotes a monovalent hydrocarbon radical which has 1 to 18 carbon atoms and may contain one or more heteroatoms from the group of N, P, S, O, and halogen,
$R^2$ denotes an SiC-bonded, divalent linear or branched hydrocarbon radical having 3 to 18 carbon atoms, preferably an alkylene radical having 3 to 10 carbon atoms,
$R^3$ denotes a hydrogen atom, an alkyl radical having 1 to 8 carbon atoms or an acyl radical, such as acetyl radical, and preferably is a hydrogen atom,
$R^4$ denotes a divalent hydrocarbon radical having 1 to 6 carbon atoms, preferably an alkylene radical having 1 to 6 carbon atoms,
k1 is 0, 1, 2 or 3,
k2 is 0, 1, 2 or 3,
x is 0, 1, 2, 3 or 4, preferably 0 or 1,
$Z^1$ is -OH, H or -NHR$^3$,
$Z^2$ is -OH, H or -NHR$^3$,

with the proviso that the sum k1 + k2 is $\geq$ 1 and that at least one radical $Z^1$ or $Z^2$ is a hydroxyl or NHR$^3$ group, preferably a hydroxyl group, and so the bridging group Y contains at least one hydroxyl or NHR$^3$ group, preferably at least one hydroxyl group.

2. Aqueous dispersions according to Claim 1, **characterized in that** the precrosslinked organopolysiloxanes comprise siloxane units of the formula

$$R_{3-d}(OR^1)_dSiO_{1/2} \qquad (III),$$

where

R has the definition indicated for it in Claim 1,
$R^1$ may be identical or different and denotes a hydrogen atom or an alkyl radical which has 1 to 18 carbon atoms and which may be interrupted by one or more separate oxygen atoms, and
d is 0 or 1.

3. Aqueous dispersions according to Claim 1 or 2, **characterized in that** Y is a radical of the formula

$$-R^2-[NH-CH_2CH_2-]_{x'}-NH\_OC-CH(OH)-CH(OH)-CO-NH-[CH_2CH_2-NH]_{x'}-R^2-$$

where

x' is 0 or 1 and
$R^2$ is a radical of the formula - $(CH_2)_3$- or -CH$_2$-CH(CH$_3$)-CH$_2$-.

4. Process for producing the aqueous dispersions, preferably aqueous emulsions, of precrosslinked organopolysiloxanes, by reacting

aqueous dispersions, preferably aqueous emulsions, of aminoorganopolysiloxanes (1) of the formula

$$(R^1O)_aA_eR_{3-a-e}SiO(SiARO)_p(SiR_2O)_qSiR_{3-d-e}A_e(OR^1)_d \qquad (IV),$$

where

Ais an amino radical of the general formula

$$-R^2-[NR^3-R^4-]_x NR^3_2 ,$$

R may be identical or different and denotes a monovalent hydrocarbon radical which has 1 to 18 carbon atoms and may contain one or more heteroatoms from the group of N, P, S, O, and halogen,

$R^1$ may be identical or different and denotes a hydrogen atom or an alkyl radical which has 1 to 18 carbon atoms and may be interrupted by one or more separate oxygen atoms,

$R^2$ denotes an SiC-bonded, divalent linear or branched hydrocarbon radical having 3 to 18 carbon atoms, preferably an alkylene radical having 3 to 10 carbon atoms,

$R^3$ denotes a hydrogen atom, an alkyl radical having 1 to 8 carbon atoms or an acyl radical, such as acetyl radical, and preferably is a hydrogen atom,

$R^4$ denotes a divalent hydrocarbon radical having 1 to 6 carbon atoms, preferably an alkylene radical having 1 to 6 carbon atoms,

d is 0 or 1,

e is 0 or 1,

p is an integer of at least 1 and at most 1000, and

q is 0 or an integer from 1 to 2000,

x is 0, 1, 2, 3 or 4, preferably 0 or 1,

with reactive esters (2) of the formula

$$R^5O_2C-[C(Z^1)(H)]_{k1}-[C(Z^2)(H)]_{k2}-CO_2R^5... \qquad (V),$$

where

$R^5$ may be identical or different and denotes an O-bonded, saturated or unsaturated, linear or branched, monovalent hydrocarbon radical which has 1-20 carbon atoms per radical and may contain one or more heteroatoms from the group of N, P, S, O and halogen,

k1 is 0, 1, 2 or 3,

k2 is 0, 1, 2 or 3,

$Z^1$ is -OH, H or -NHR$^3$,

$Z^2$ is -OH, H or -NHR$^3$,

with the proviso that the sum k1 + k2 is $\geq$ 1 and that at least one radical $Z^1$ or $Z^2$ is a hydroxyl or NHR$^3$ group, preferably a hydroxyl group.

5.  Process according to Claim 4, **characterized in that** A is an amino radical of the formula

$$-R^2-[NH-CH_2CH_2-]_{x'}-NH_2$$

where

x' is 0 or 1 and

$R^2$ is a radical of the formula $-(CH_2)_3-$ or $-CH_2-CH(CH_3)-CH_2-$.

6.  Process according to Claim 4 or 5, **characterized in that** the reactive esters (2) are dimethyl tartrates and diethyl tartrates.

7.  Precrosslinked organopolysiloxanes containing on average at least one structural unit, preferably at least two structural units, more preferably at least three structural units, of the general formula

$$SiRO_{2/2}-Y-SiRO_{2/2} \qquad (I)$$

and units of the formula

$$R_2SiO_{2/2} \qquad (II),$$

where

Yis a radical of the formula

$$-R^2-[NR^3-R^4-]_x-NR^3-OC-[C(Z^1)(H)]_{k1}-[C(Z^2)(H)]_{k2}-CO-NR^3-[R^4-NR^3]_x-R^2-,$$

R, $R^2$, $R^3$, $R^4$, k1, k2, $Z^1$ and $Z^2$ have the definition indicated for them in Claim 1,
with the proviso that the sum k1 + k2 is $\geq$ 1 and that at least one radical $Z^1$ or $Z^2$ is a hydroxyl or $NHR^3$ group, preferably a hydroxyl group, and so the bridging group Y contains at least one hydroxyl or $NHR^3$ group, preferably at least one hydroxyl group.

8. Precrosslinked organopolysiloxanes according to Claim 7, **characterized in that** they comprise siloxane units of the formula

$$R_{3-d}(OR^1)_dSiO_{1/2} \qquad (III)$$

where

R and Y have the definition indicated for them in Claim 1 and
d and $R^1$ have the definition indicated for them in Claim 2.

9. Process for producing the precrosslinked organopolysiloxanes, **characterized in that** aminoorganopolysiloxanes of the formula

$$(R^1O)_aA_eR_{3-a-e}SiO(SiARO)_p(SiR_2O)_qSiR_{3-d-e}A_e(OR^1)_d \qquad (IV)$$

are reacted with reactive esters of the formula

$$R^5O_2C-[C(Z^2)(H)]_{k1}-[C(Z^2)(H)]_{k2}-CO_2R^5... \qquad (V)$$

where

A, R, $R^1$, $R^5$, k1, k2, $Z^1$ and $Z^2$ have the definitions indicated for them in Claim 4,
with the proviso that the sum k1 + k2 is $\geq$ 1 and that at least one radical $Z^1$ or $Z^2$ is a hydroxyl or $NHR^3$ group, preferably a hydroxyl group,
and the resulting precrosslinked organopolysiloxanes are optionally subsequently emulsified in water.

10. Cosmetic compositions comprising aqueous dispersions, preferably aqueous emulsions, of precrosslinked organopolysiloxanes according to Claim 1, 2 or 3 or produced according to Claim 4, 5 or 6, or precrosslinked organopolysiloxanes according to Claim 7 or 8 or produced according to Claim 9.

11. Cosmetic compositions according to Claim 10, **characterized in that** they comprise conditioning agents.

12. Use of the cosmetic compositions according to Claim 10 or 11 for treating keratinic fibers, preferably for cleansing and caring for keratinic fibers.

13. Use of the cosmetic compositions according to Claim 10 or 11 for conditioning keratinic fibers, more particularly for facilitating the combability of keratinic fibers.

14. Use according to Claim 12 or 13, **characterized in that** the keratinic fibers are hair.

15. Process for treating keratinous fibers, preferably hair, by applying the cosmetic compositions according to Claim 10 or 11 to the keratinic fibers, preferably hair, and then optionally rinsing with water.

**Revendications**

1. Dispersions aqueuses, de préférence émulsions aqueuses, contenant des organopolysiloxanes pré-réticulés, qui contiennent en moyenne au moins un motif structural de formule générale

$$SiRO_{2/2}-Y-SiRO_{2/2} \qquad (I)$$

et des motifs de formule

$$R_2SiO_{2/2} \qquad (II),$$

dans lesquelles

Y représente un radical de formule $-R^2-[NR^3-R^4-]_x-NR^3-OC-[C(Z^1)(H)]_{k1}-[C(Z^2)(H)]_{k2}-CO-NR^3-[R^4-NR^3]_x-R^2$

R peut être identique ou différent, représente un radical hydrocarboné monovalent ayant 1 à 18 atomes de carbone, qui peut contenir un ou plusieurs hétéroatomes du groupe N, P, S, O et halogène,

$R^2$ représente un radical hydrocarboné linéaire ou ramifié, divalent, lié à SiC, ayant 3 à 18 atomes de carbone, de préférence un radical alkylène ayant 3 à 10 atomes de carbone,

$R^3$ représente un atome d'hydrogène, un radical alkyle ayant 1 à 8 atomes de carbone ou un radical acyle, tel que le radical acétyle, et représente de préférence un atome d'hydrogène,

$R^4$ représente un radical hydrocarboné divalent ayant 1 à 6 atomes de carbone, de préférence un radical alkylène ayant 1 à 6 atomes de carbone,

k1 représente 0, 1, 2 ou 3,

k2 représente 0, 1, 2 ou 3,

x représente 0, 1, 2, 3 ou 4, de préférence 0 ou 1,

$Z^1$ représente -OH, H ou $-NHR^3$,

$Z^2$ représente -OH, H ou $-NHR^3$,

à la condition que la somme k1 + k2 soit ≥ 1 et qu'au moins un radical $Z^1$ ou $Z^2$ représente un groupe hydroxy ou $NHR^3$, de préférence un groupe hydroxy, le groupe pontant Y contenant ainsi au moins un groupe hydroxy ou $NHR3$, de préférence au moins un groupe hydroxy.

2. Dispersions aqueuses selon la revendication 1, **caractérisées en ce que** les organopolysiloxanes pré-réticulés contiennent des motifs siloxane de formule

$$R_{3-a}(OR^1)_dSiO_{1/2} \qquad (III),$$

dans laquelle

R a les significations qui lui sont données dans la revendication 1,

$R^1$ peut être identique ou différent, représente un atome d'hydrogène ou un radical alkyle ayant 1 à 18 atomes de carbone, qui peut être interrompu par un ou plusieurs atomes d'hydrogène séparés, et

d représente 0 ou 1.

3. Dispersions aqueuses selon la revendication 1 ou 2, **caractérisées en ce que** Y représente un radical de formule

$$-R^2-[NH-CH_2CH_2-]_{x'}-NH-OC-CH(OH)-CH(OH)-CO-NH-[CH_2CH_2-NH]_{x'}-R^2-$$

dans laquelle

x' représente 0 ou 1 et

$R^2$ représente un radical de formule $-(CH_2)_3-$ ou $-CH_2-CH(CH_3)-CH_2-$.

4. Procédé de fabrication des dispersions aqueuses, de préférence d'émulsions aqueuses, d'organopolysiloxanes pré-réticulés, dans lequel on fait réagir

des dispersions aqueuses, de préférence des émulsions aqueuses d'aminoorganopolysiloxanes (1) de formule

$$(R^1O)_aA_eR_{3-a-e}SiO(SiARO)_p(SiR_2O)_qSiR_{3-d-e}A_e(OR^1)_d \qquad (IV)$$

dans laquelle

A représente un radical amino de formule générale

$$-R^2-[NR^3-R^4-]_xNR^3_2,$$

R peut être identique ou différent, représente un radical hydrocarboné monovalent ayant 1 à 18 atomes de carbone, qui peut contenir un ou plusieurs hétéroatomes du groupe N, P, S, O et halogène,

$R^1$ peut être identique ou différent, représente un atome d'hydrogène ou un radical alkyle ayant 1 à 18 atomes de carbone, qui peut être interrompu par un ou plusieurs atomes d'oxygène séparés,

$R^2$ représente un radical hydrocarboné linéaire ou ramifié, divalent, lié à SiC, ayant 3 à 18 atomes de carbone, de préférence un radical alkylène ayant 3 à 10 atomes de carbone,

$R^3$ représente un atome d'hydrogène, un radical alkyle ayant 1 à 8 atomes de carbone ou un radical acyle, tel que le radical acétyle, et représente de préférence un atome d'hydrogène,

$R^4$ représente un radical hydrocarboné divalent ayant 1 à 6 atomes de carbone, de préférence un radical alkylène ayant 1 à 6 atomes de carbone,

d représente 0 ou 1,

e représente 0 ou 1,

p représente un nombre entier valant au moins 1 et au plus 1 000 et

q représente 0 ou un nombre entier de 1 à 2 000,

x représente 0, 1, 2, 3 ou 4, de préférence 0 ou 1,

avec des esters réactifs (2) de formule

$$R^5O_2C-[C(Z^1)(H)]_{k1}-[C(Z^2)(H)]_{k2}-CO_2R^5... \qquad (V),$$

dans laquelle

$R^5$ peut être identique ou différent et représente un radical hydrocarboné monovalent, linéaire ou ramifié, saturé ou insaturé, lié à O, ayant 1 à 20 atomes de carbone par radical, qui peut contenir un ou plusieurs hétéroatomes du groupe N, P, S, O et halogène,

k1 représente 0, 1, 2 ou 3,

k2 représente 0, 1, 2 ou 3,

$Z^1$ représente -OH, H ou -NHR$^3$,

$Z^2$ représente -OH, H ou -NHR$^3$,

à la condition que la somme k1 + k2 soit $\geq$ 1 et qu'au moins un radical $Z^1$ ou $Z^2$ représente un groupe hydroxy ou NHR$^3$, de préférence un groupe hydroxy.

**5.** Procédé selon la revendication 4, **caractérisé en ce que** A représente un radical amino de formule

$$-R^2-[NH-CH_2CH_2-]_{x'}-NH_2$$

dans laquelle

x' représente 0 ou 1 et

$R^2$ représente un radical de formule - $(CH_2)_3$- ou -CH$_2$-CH(CH$_3$)-CH$_2$-.

**6.** Procédé selon la revendication 4 ou 5, **caractérisé en ce que** les esters réactifs (2) sont des tartrates de diméthyle ou des tartrates de diéthyle.

**7.** Organopolysiloxanes pré-réticulés, qui contiennent en moyenne au moins un motif structural, de préférence au moins deux motifs structuraux, de préférence au moins trois motifs structuraux, de formule générale

$$SiRO_{2/2}-Y-SiRO_{2/2} \qquad (I)$$

et des motifs de formule

$$R_2SiO_{2/2} \qquad (II),$$

dans lesquelles

Y représente un radical de formule

$$-R^2-[NR^3-R^4-]_x-NR^3\_OC-[C(Z^1)(H)]_{k1}-[C(Z^2)(H)]_{k2}-CO-NR^3-[R^4-NR^3]_x-R^2-,$$

R, $R^2$, $R^3$, $R^4$, k1, k2, $Z^1$ et $Z^2$ ont les significations qui leur sont données dans la revendication 1,
à la condition que la somme k1 + k2 soit $\geq$ 1 et qu'au moins un radical $Z^1$ ou $Z^2$ représente un groupe hydroxy ou $NHR^3$, de préférence un groupe hydroxy, le groupe pontant Y contenant ainsi au moins un groupe hydroxy ou NHR3, de préférence au moins un groupe hydroxy.

8. Polyorganosiloxanes pré-réticulés selon la revendication 7, **caractérisés en ce qu'**ils contiennent des motifs siloxane de formule

$$R_{3-a}(OR^1)_d SiO_{1/2} \qquad (III)$$

dans laquelle

R et Y ont les significations qui leur sont données dans la revendication 1 et
d et $R^1$ ont les significations qui leur sont données dans la revendication 2.

9. Procédé de fabrication des organopolysiloxanes pré-réticulés, **caractérisé en ce qu'**on fait réagir des aminoorganopolysiloxanes de formule

$$(R^1O)_a A_e R_{3-a-e} SiO(SiARO)_p(SiR_2O)_q SiR_{3-d-e} A_e(OR^1)_d \qquad (IV)$$

avec des esters réactifs de formule

$$R^5O_2C-[C(Z^1)(H)]_{k1}-[C(Z^2)(H)]_{k2}-CO_2R^5... \qquad (V)$$

dans lesquelles

A, R, $R^1$, $R^5$, k1, k2, $Z^1$ et $Z^2$ ont les significations qui leur sont données dans la revendication 4,
à la condition que la somme k1 + k2 soit $\geq$ 1 et qu'au moins un radical $Z^1$ ou $Z^2$ représente un groupe hydroxy ou NHR3, de préférence un groupe hydroxy,
puis on émulsifie éventuellement les organopolysiloxanes pré-réticulés obtenus.

10. Préparations cosmétiques contenant des dispersions aqueuses, de préférence des émulsions aqueuses, d'organopolysiloxanes pré-réticulés selon la revendication 1, 2 ou 3 ou fabriquées selon la revendication 4, 5 ou 6, ou des organopolysiloxanes pré-réticulés selon la revendication 7 ou 8 ou fabriqués selon la revendication 9.

11. Compositions cosmétiques selon la revendication 10, **caractérisées en ce qu'**elles contiennent des agents conditionnants.

12. Utilisation des compositions cosmétiques selon la revendication 10 ou 11 pour traiter des fibres kératiniques, de préférence pour le nettoyage et le soin de fibres kératiniques.

13. Utilisation des compositions cosmétiques selon la revendication 10 ou 11 pour conditionner des fibres kératiniques, en particulier pour faciliter la peignabilité de fibres kératiniques.

14. Utilisation selon la revendication 12 ou 13, **caractérisée en ce que** les fibres kératiniques sont des cheveux.

15. Procédé de traitement de fibres kératiniques, de préférence de cheveux, dans lequel on applique les compositions cosmétiques selon la revendication 10 ou 11 sur les fibres kératiniques, de préférence les cheveux, puis on les rince éventuellement à l'eau.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 7223385 B2
- US 7485289 B2
- US 7220408 B2
- US 7504094 B2
- US 5039738 A
- WO 2015024079 A1
- WO 2004039930 A2
- US 5399652 A
- US 8609787 B2
- US 7129369 B2

**In der Beschreibung aufgeführte Nicht-Patentliteratur**

- Monograph. **M.D. BERTHIAUME.** Silicones in Hair Care. Society of Cosmetic Chemists (Hrsg.), 1997
- **J. SEJPKA.** Silicone in Haarpflegeprodukten. *SÖFW-Journal,* 1992, (17), 1065-1070
- Wetting Properties of Human Hair by Means of Dynamic Contact Angle Measurement. **R.A. LODGE ; B. BHUSAN.** Journal of Applied Polymer Science. Wiley, 2006, vol. 102, 5255-5265
- **K. KRUMMEL ; STEPHANE CHIRON ; J. JACHOWICZ.** *The Chemistry and Manufacture of Cosmetics,* 2000, 359-396
- International Cosmetic Ingredient Dictionary & Handbook. The Cosmetic, Toiletry, and Fragrance Association (CTFA), 2010
- **K. SCHRADER ; A. DOMSCH.** Cosmetology - Theory and Practice. Verlag, 2005, vol. II, II-8-II-22
- **Y. K. KAMATH ; HANS-DIETRICH WEIGMANN.** *J. Soc. Cosmet. Chem.,* 1986, vol. 37, 111-124